# EUROPEAN PATENT SPECIFICATION

(11) **EP 1 594 569 B1**
(45) Date of publication and mention of the grant of the patent: **14.05.2014**
(21) Application number: 04705148.7
(22) Date of filing: 26.01.2004
(51) Int. Cl.: A61N 1/362, A61F 2/00

(54) **IN VIVO DEVICE FOR IMPROVING DIASTOLIC VENTRICULAR FUNCTION**
IN VIVO VORRICHTUNG ZUR VERBESSERUNG DER DIASTOLISCHEN VENTRIKELFUNKTION
DISPOSITIF IN VIVO DESTINE A AMELIORER LA FONCTION VENTRICULAIRE DIASTOLIQUE

(30) Priority: 27.01.2003 IL 15414103; 03.11.2003 US 516272 P
(43) Date of publication of application: 16.11.2005
(60) Divisional application: 12162908.3
(73) Proprietor: Corassist Cardiovascular Ltd., 46733 Herzliya (IL)
(72) Inventor: DUBI, Shay, Tel Aviv (IL); FELD, Yair, 34371 Haifa (IL)
(74) Representative: Vossius & Partner
(86) International application number: PCT/IL2004/000072
(87) International publication number: WO 2004/066805

(56) References cited:
- WO-A-01/91667
- WO-A-02/19917
- WO-A-99/53977
- US-B1- 6 264 602

## Description

### Field of the Invention

The present invention relates to a device for improving ventricular function of the heart and, more particularly, to a modified in vivo device for improving diastolic function of the left ventricle of the heart.

### Background of the Invention

Heart failure is commonly defined as the inability of the left ventricle, herein, also referred to as LV, to generate an adequate cardiac output at rest or during exertion, while operating at a normal or enhanced LV filling pressure. Congestive heart failure (CHF) is a clinical syndrome in which heart failure is accompanied by the symptoms and signs of pulmonary and/or peripheral congestion. Heart failure is most commonly associated with impaired LV systolic function. A widely used index for quantifying systolic function is 'ejection fraction' (EF), defined as the ratio of stroke volume to end-diastolic volume, which can be estimated using techniques such as radiocontrast, radionuclide angiography, and/or, echocardiography. The normal value of EF is 0.67 ± 0.08, which is frequently depressed in systolic heart failure even when the stroke volume is normal. A value of EF ≥ 0.50 is commonly used as an indicator of normal systolic function. It is notable, however, that as much as 30 - 50 % of all patients with typical symptoms of congestive heart failure have a normal or slightly reduced ejection fraction, that is, a value of EF ≥ 0.45.

In these patients, diastolic dysfunction is implicated as a major contributor of congestive heart failure. In some patients, systolic and diastolic heart failure coexist. The most common form of heart failure, the one caused by coronary arteriosclerosis, is an example of combined systolic and diastolic failure, as described in "Braunwald's Heart Disease: Review and Assessment", third edition, 1997, Saunders Company Publishers. There are about 4.6 million people in the United States with heart failure, and about 550,000 are being reported annually, as indicated by Vasan, R.S., and Benjamin, E.J., in "Diastolic Heart Failure - No Time to Relax", New England Journal of Medicine 2001, 344: 5.6 - 59. Also indicated therein, is that the mortality rate from diastolic heart failure (DHF), 5 - 12 % annually, is about four times that among persons without heart failure and half that among patients with systolic heart failure, and that, nonetheless, rates of hospitalization and health care associated with diastolic heart failure rival those associated with systolic heart failure.

Primary diastolic dysfunction is typically observed in patients with hypertension and hypertrophic or restrictive cardiomyopathy, but can also occur in a variety of other clinical disorders and has a particularly high prevalence in the elderly population. Aging is associated with 'physiologic' diastolic dysfunction due to the increase in LV muscle mass and changes in passive elastic properties of the myocardium, hence, the concern of an increase in the incidence of diastolic dysfunction as the aging of the western world population progresses.

For the purpose of clearly understanding, and implementing, the following described preferred embodiments of the present invention, relevant details, description, and, definitions of selected terms, well known to one of ordinary skill in the art, of physiological and pathological aspects, mechanisms, and functions, of the heart, in general, and of the ventricles and atria, in particular, are provided herein. Additional details, description, and, definitions of terms, thereof, are readily available in the scientific literature.

The left ventricle is the chamber on the left side of the heart that receives oxygenated arterial blood from the left atrium and contracts to drive it into the aorta for distribution to the body. The right ventricle is the chamber on the right side of the heart that receives deoxygenated venous blood from the right atrium and drives it into the pulmonary artery in order to receive oxygen from the lungs. Diastole is the normal rhythmically occurring relaxation and dilatation (stretching, expansion, dilation) of the heart cavities (ventricles), during which the cavities are filled with blood. Atrial contraction occurs during the last stage of diastole of the ventricle and aids ventricular filling. Systole is the rhythmic contraction of the heart, especially of the ventricles, by which blood is driven through the aorta and pulmonary artery after each dilation or diastole.

Ventricular filling starts just after mitral valve opening. As the LV pressure decreases below that in the left atrium, the phase of rapid or early filling of the LV accounts for most of ventricular filling. LV filling temporarily stops as pressures in the atrium and left ventricle equalize, commonly known as the phase of diastasis, occurring prior to atrial contraction and during which little blood enters the filled left ventricle. Atrial contraction increases the pressure gradient from the atrium to the left ventricle to renew filling. When the LV fails to relax normally, as in 'LV hypertrophy', increased atrial contraction can enhance late filling. Relaxation (inactivation of contraction) is a dynamic process that begins at the termination of contraction and occurs during isovolumetric relaxation and early ventricular filling. 'Myocardial elasticity' is the change in muscle length for a given change in force. 'Ventricular compliance' is the change in ventricular volume for a given change in pressure, and, 'ventricular stiffness' is the inverse of compliance.

The 'preload' is the load present before contraction has started and is provided by the venous return that fills the ventricle during diastole. The 'Frank Starling law of the heart' states that the larger the volume of the heart, the greater the energy of its contraction and hence the stroke volume is larger. In other words, when the preload increases, the left ventricle distends (widens, expands) and the stroke volume increases, as described by Opie, H.L., in "The Heart Physiology, From Cell To Circulation", third edition, Lippincott-Raven publishers, 1998. The pressure-volume relation curves are an accepted description of the ventricular function.

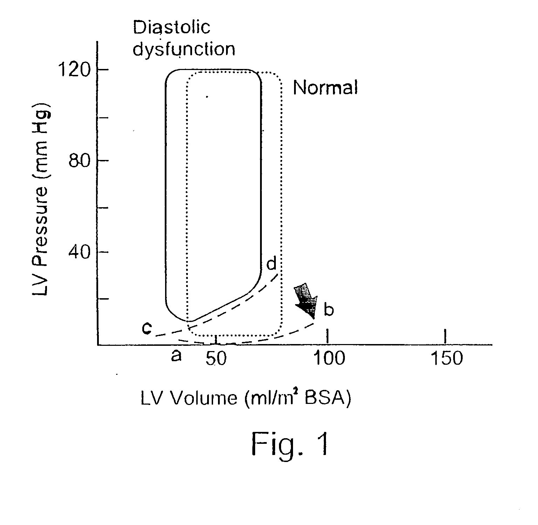

FIG. 1, adapted from the previously cited "Braunwald's Heart Disease: Review and Assessment" reference, is a schematic diagram illustrating a typical pressure-volume loop of a normal subject (dotted line) and a patient with diastolic dysfunction (solid line), wherein dashed lines, between the letters 'a' and 'b', and, 'c' and 'd', represent the diastolic pressure-volume relation of the normal subject, and, the patient with diastolic dysfunction, respectively. FIG. 1 shows that isolated diastolic dysfunction is characterized by a shift in the pressure-volume loop to the left. Contractile performance is normal, associated with an ejection fraction (EF) value ≥ 0.45, with a normal or slightly decreased stroke volume. However, LV (left ventricular) pressures throughout diastole are increased, at a common diastolic volume equal to about 70 ml/m². In the patient with diastolic failure, LV end diastolic pressure is about 25 mm Hg, compared with an LV end diastolic pressure of about 5 mm Hg in the normal subject. Thus, diastolic dysfunction increases the modulus of chamber stiffness. A main objective of treating the patient with diastolic dysfunction is to cause the diastolic pressure-volume relation curve (dashed line between 'c' and 'd') to go back to the diastolic pressure-volume relation curve (dashed line between 'a' and 'b', also indicated by the arrow), of the normal subject, by decreasing the end diastolic LV pressure for the same LV volume.

The fundamental problem in diastolic heart failure (DHF) is the inability of the left ventricle to accommodate blood volume during diastole at low filling pressures, as described by Mandinov, L., Eberli, F.R., Seiler, C., and Hess, M.O., in "Diastolic Heart Failure", Cardiovascular Res. 2000, 45: 813 - 825. Initially, hemodynamic changes may be manifested only in an upward displacement of the diastolic pressure-volume curve in the presence of a normal end-diastolic volume with inappropriate elevation of LV diastolic, left atrial and pulmonocapillary pressure (as previously described above, with reference to FIG. 1). More severe resistance to LV filling may cause inadequate filling even in enhanced diastolic pressure with an additional leftward shift of the diastolic pressure-volume relation, resulting in a decreased end diastolic volume and depressed stroke volume, as described by Mandinov, L., et al.

Currently, four different pathophysiological mechanisms are known and used for understanding and/or explaining diastolic heart failure (DHF), combinations of which may readily take place in a particular patient: (1) slow isovolumic left ventricular relaxation, (2) slow early left ventricular filling, (3) reduced left ventricular diastolic distensibility, and, (4) increased left ventricular chamber stiffness or increased myocardial muscle stiffness, as described in the report, "How To Diagnose Diastolic Heart Failure: European Study Group On Diastolic Heart Failure", European Heart Journal, 1998, 19: 990 - 1003.

Slow isovolumic left ventricular relaxation, (1), refers to a longer time interval between aortic valve closure and mitral valve opening and a lower negative peak ventricular dP/dt. Regional variation in the onset, rate, and extent of myocardial lengthening is referred to as 'diastolic asynergy'; temporal dispersion of relaxation, with some fibers commencing to lengthen later than others, is referred to as 'asynchrony'. Slow early left ventricular filling, (2), is a result of slow myocardial relaxation, segmental incoordination related to coronary artery disease and the atrioventricular pressure gradient. Reduced left ventricular diastolic distensibility, (3), refers to an upward shift of the LV pressure-volume relation on the pressure-volume plot, irrespective of a simultaneous change in slope. Reduction in LV end diastolic distensibility is usually caused by extrinsic compression of the ventricles as in cardiac tamponade. Increased LV chamber stiffness or increased myocardial muscle stiffness, (4), as manifested by a shift to a steeper ventricular pressure-volume curve, is due to processes such as ventricular hypertrophy, endomyocardial fibrosis, disorders with myocardial infiltration (for example, amyloidosis) and replacement of normal, distensible myocardium with non-distensible fibrous scar tissue in healed infarct zones.

The previously cited European Study Group proposed criteria for the diagnosis of DHF. Accordingly, simultaneous presence of the following three criteria is considered obligatory for establishing a diagnosis of DHF: (1) evidence of CHF, (2) normal or mildly abnormal LV systolic function, (3) evidence of abnormal LV relaxation, filling, diastolic distensibility, or, diastolic stiffness.

Pulmonary edema is the result of the increase in pulmocapillary pressure and is due to a shift of liquid from the intravascular compartment to the lung interstitial compartment. Pulmonary edema is frequently associated with hypertension. Gandhi, S.K. et al., in "The Pathogenesis Of Acute Pulmonary Edema Associated With Hypertension", New England Journal of Medicine, 2001, 344: 17 - 22, have contradicted the hypothesis that pulmonary edema, apparently associated with hypertension, in patients with preserved ejection fraction, is due to transient systolic dysfunction. They found that the LV ejection fraction and the extent of regional wall motion measured during the acute episode of hypertensive pulmonary edema were similar to those measured after the resolution of the congestion, when the blood pressure was controlled, thus concluding that the pulmonary edema was due to diastolic rather than systolic heart failure.

The management of diastolic heart failure is difficult. There have been no large-scale, randomized controlled trials of therapy in diastolic heart failure, and there remains substantial disagreement about the appropriate therapy for this disease, according to Sweitzer, N.K., and Stevenson, L.W., in "Diastolic heart Failure: Miles To Go Before We Sleep", American Journal of Medicine, 2000, 109: 683 - 685. Medical therapy of diastolic dysfunction is often empirical and lacks clear-cut pathophysiologic concepts, as indicated in previously cited Mandinov, L. et al.. No single drug presently exists which selectively enhances myocardial relaxation without negative effects on LV contractility or pump function, and thus, there is a significant need for a new therapeutic approach for this particular type of heart disease.

Treatment of diastolic heart failure may be logically divided into three areas or categories: (1) removal of the precipitating cause, (2) correction of the underlying cause, and, (3) control of the congestive heart failure state. Treatment goals that have been advocated, by previously cited Mandinov, L. et al., and, by Braunwald, E., in "Heart Failure", Harrison's Principles of Internal Medicine, fourteenth edition, McGraw Hill publishers, are as follows:
1. *Reduction of central blood volume*. Reduction of salt intake and use of diuretics (usually, loop diuretics). Diuretics are effective in reducing pulmonary congestion, shifting the pressure-volume relation downwards. However, they must be used with care because the volume sensitivity of patients with diastolic dysfunction bears the risk that excessive diuresis may result in a sudden drop in stroke volume. Because of the steep pressure-volume relationship, a small decrease in diastolic volume will cause a large decrease of the filling pressure, and will result in a drop in stroke volume, and thus, in cardiac output.
2. *Reduction of workload.* Reduction of physical activity, maintenance of emotional rest and use of vasodilators. Vasodilators, such as sodium nitroprusside or ACE inhibitors reduce the filling pressure and the afterload in all patients, and elevate cardiac output. Reduction of an elevated left ventricular end diastolic pressure may improve subendocardial perfusion, thus improving myocardial contraction. Nonetheless, vasodilators have not been useful in the management of isolated diastolic heart failure and are more effective in combined heart failure, as indicated in the previously cited Braunwald, E. text. Vigorous control of hypertension is imperative in patients with heart failure caused by diastolic dysfunction, because control of hypertension may prevent progression or may partially reverse the disorder by addressing the primary cause of most cases, as described by Grauner, K., in "Heart Failure, Diastolic Dysfunction and the Role of the Family Physician", American Family Physician, 2001, 63: 1483 - 1486.
3. *Improvement of LV relaxation.* In particular, by using calcium channel blockers or ACE inhibitors. Ca²⁺ channel blockers have been shown to improve myocardial relaxation and enhance diastolic filling. These drugs may be best matched to the pathophysiology of relaxation disturbances due to their ability to decrease cytoplasmic calcium concentration and reduce afterload. However, currently, use of Ca²⁺ channel blockers is limited due to their negative inotropic effects (negative influence on the systolic function of the heart), and clinical trials have not clearly proven them to be beneficial.
4. *Regression of LV hypertrophy.* In particular, decrease in wall thickness and removal of excess collagen by ACE inhibitors and AT-2 antagonists or Spironolactone. Philbin, E.F., Rocco, T.A., Lindenmuth, N.W., Ulrich, K., and Jenkins, O.L., in "Systolic Versus Diastolic Heart Failure In Community Practice: Clinical Features, Outcomes, And The Use Of ACE Inhibitors", American Journal of Medicine, 2000, 109: 605 - 613, have shown that the use of ACE inhibitors in patients with ejection fraction equal to or greater than 0.50 was associated with a better NYHA class (New York Heart Association functional and therapeutic classification for stages of heart failure) after discharge from hospitalization, but had no significant effect on mortality or hospital readmission. ACE inhibitors and AT-2 antagonists affect blood pressure, reduce afterload, and affect the myocardium via the local renin-angiotensin system. These effects are important for regression of LV hypertrophy, and improvement of elastic properties of the myocardium.
5. *Maintenance of atrial contraction and control of heart rate*. In particular, by using beta-blockers and/or antiarrhythmics. Beta-blockers reduce blood pressure and myocardial hypertrophy. The positive effect on diastolic dysfunction is mainly due to slowing of the heart rate and not to a primary improvement in isovolumic relaxation or the diastolic properties of the left ventricle.
6. *NO donors.* NO (Nitric Oxide) donors have been shown to exert a relaxant effect on the myocardium, which is associated with a decrease in LV end diastolic pressure. In patients with severe LV, hypertrophy, an increased susceptibility to NO donors has been documented, which may be beneficial for the prevention of diastolic dysfunction.
7. *Heart transplantation*. Heart transplantation is a definitive treatment for end stage heart failure.
8. *Biventricular pacing.* Biventricular pacing improves uncoordinated contraction due to left bundle branch block or other conduction abnormalities with wide 'QRS complex' (P-Q-R-S-T waveform) of an electrocardiogram, which are common in patients with CHF. Morris-Thurgood, J.A., Turner, M.S., Nightingale, A.K., Masani, N., Mumford, C., and, Frenneaux, M.P., in "Pacing In Heart Failure: Improved Ventricular Interaction In Diastole Rather Than Systolic Re-synchronization", Europace 2000, 2: 271 - 275, have shown that left ventricular pacing acutely benefits congestive heart failure patients with pulmonary capillary wedge pressure greater than 15 mm Hg, irrespective of left bundle branch block. They suggested the beneficial mechanism might be related to an improvement of ventricular interaction in diastole (VID) rather than ventricular systolic re-synchronization. According to their suggestion, LV pacing in patients with high LV end diastolic pressure, will delay right ventricular filling and allow greater LV filling before the onset of VID. Biventricular pacing, however, has not been clinically proven effective in the treatment of patients with diastolic heart failure.

WO 02/19917 describes a device designed for reducing the diameter of the heart by means of a heart support structure designed to transfer energy, in the form of contraction and expansion, from viable heart tissue to less viable or non-viable heart tissue. The device is constructed from support links that extend from a central point and snake towards peripheral links. A desired stiffness profile of the support structure is required in order to determine the degree of contraction and expansion transferred from viable tissue to injured tissue throughout the support structure. WO 01/91667 describes devices and methods for reconfiguring one or more chambers of a natural heart. The device in this publication is comprised of bundles of spring wires configured to lie against the inner walls of a ventricle and apply an average outward force on the ventricle. The spring wires are joint at a junction point forming a post tip merging into a solid rod.

US 6,264,602 describes a device for changing chamber geometry employing splints and wraps which are placed around the heart for limiting muscle stress without causing chamber shape change. The device in this publication may be configured in a form of a Band splint. In one of its embodiments the device includes a horizontally heart encircling band and four bands extending downward therefrom to a second horizontally heart encircling band.

To one of ordinary skill in the art, there is thus a need for, and it would be highly advantageous to have an in vivo device for use in improving diastolic function of the left ventricle of the heart, while minimally disturbing systolic function of the heart. Moreover, there is a need for such a device which is biocompatible and is specially configured for compact and long-term reliable use in humans.

One of the purposes of the present invention is to provide an indwelling *in vivo* device that may be used to improve diastolic function of either the left ventricle or right ventricle of the heart.

Another purpose of the present invention is to provide such a device that may be readily adapted to the precise topographic conformation of the heart that is to be treated.

Yet another purpose of the present invention is to provide such a device that may be readily delivered to the required site on the external surface of the ventricle by non-invasive or minimally-invasive means.

A further purpose of the present invention is to provide an *in vivo* device that overcomes the problems and disadvantages of previous devices.

Further objects and advantages of the present invention will become clear as the description proceeds.

### SUMMARY OF THE INVENTION

The present invention is defined by the appended claims. It relates to an in vivo device for improving diastolic function of the left or right ventricle of the heart, said device being a modification of the devices disclosed in WO 03/007778 A2.

The present invention is primarily directed to an anatomically-compatible and physiologically-compatible *in vivo* device for improving diastolic function of either the left or right ventricle of the heart, comprising:
at least one elastic component that is capable of being operatively connected to the external ventricular surface of the heart by means of one or more connecting elements,
wherein said at least one elastic component comprises a plurality of essentially longitudinal members which are arranged such that the lateral separation between adjacent longitudinal members may be increased or decreased in response to elastic deformation of said elastic component,
and wherein said essentially longitudinal members are arranged relative to each other such that said elastic component is curved in both the vertical and horizontal planes, such that the inner surface of said elastic component may be adapted to the curvature of the external ventricular surface of the heart, or a portion thereof,
such that said at least one elastic component is capable of exerting both a radially outward expansive force and a tangentially-directed force on the external ventricular surface of the heart to which said component may be connected by means of said one or more connecting elements.

The term "anatomically compatible" as used hereinbefore refers to the fact that the structure of the device of the invention is such that it may readily be adapted *in situ* to the precise shape and size of the heart to be treated.

The term "physiologically compatible" as used hereinbefore refers to the fact that the structure of the device of the invention is such that it may readily be adapted *in situ* to the precise movement vectors of the heart to be treated.

In the device of the invention, the elastic component comprises a plurality of elongated members, each of said elongated members having one end connected to, and continuous with, a base element, said base element being of a size and shape such that it is capable of either fully or partially encircling the apical region of the heart, and wherein said elongated members are arranged such that they are capable of being disposed in an essentially longitudinal manner along the external ventricular surface of the heart, such that said free ends of said elongated members are directed towards the base of the heart. In a particularly preferred embodiment, the abovementioned base element is provided in an annular shape.

Although the at least one elastic component of the in vivo device of the invention may be constructed of any suitable material possessing the desired spring-like properties, in a preferred embodiment, said at least one elastic component is constructed from a material selected from the group consisting of tungsten, platinum, titanium, nitinol alloy, stainless steel alloy, biocompatible plastics (e.g. silicon) and, combinations thereof.

According to one preferred embodiment of the device of the invention, said device is constructed such that the aforementioned maximal value for the radially outward expansive pressure exerted on at least one part of the external ventricular wall is in a range of about 5 mm Hg to about 40 mm Hg.

As mentioned hereinabove, the *in vivo* device according to the present invention possesses a number of further significant advantageous properties in addition to those described in relation to the corresponding devices disclosed in co-pending international patent application no. PCT/IL02/00547. Among these advantages are included the following desirable properties:
a) greater anatomical compatibility of the presently-disclosed device with the left ventricle of the heart to which said device is attached;
b) greater physiological compatibility of the presently-disclosed device with the movement of the left ventricle of the heart to which said device is attached;
c) increased range of forces and/or pressures attainable with a single device;
d) increased range of left ventricular sizes that may be accommodated with a single device;
e) increased ease with which the device may be delivered to the desired region of the left ventricle by non-invasive or minimally-invasive endoscopic means;
f) greater ease of construction of the device; and
g) significantly lower cost of construction of the device.

Further properties and advantages of the presently-claimed device will become apparent as the description proceeds.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention.
FIG. 1 is a schematic diagram illustrating a typical pressure-volume loop of a normal subject and a patient with diastolic dysfunction.
FIG. 2 depicts a preferred embodiment of the *in vivo* device of the invention, in which said device comprises a plurality of elongated members connected at one end by a base element.
FIG. 3 is an illustration of a different version of the device of FIG. 2 not part of the invention.
FIG. 4 depicts the device illustrated in FIG. 3 in its *in situ* position on the external surface of the left ventricle.
FIG. 5 illustrates the use of Dacron tubes as connecting elements for attaching a device to the external surface of the heart.
FIG. 6 schematically illustrates one type of thoracoscopic delivery system that may be used for delivering a device of the present invention to its attachment site on the external surface of the ventricle wall.
FIG. 7 illustrates a v-shaped wire spring not part of the invention.
FIG. 8 depicts some examples of *in vivo* devices not part of the invention that incorporate a wire spring as their elastic element. FIG. 8A shows a device comprising a wire spring that has been bent into a series of v-shaped bends over its entire length. FIG. 8B depicts the embodiment shown in FIG. 8A in its *in situ* position on the external cardiac wall. FIG. 8C illustrates another wire spring device, in which said spring comprises two v-shaped sections separated by a linear portion. FIG. 8D illustrates the device of FIG. 8C in its *in situ* position on the external left ventricular wall.
FIG. 9 schematically illustrates the direction of the forces exerted by a device of the invention on the ventricular wall.
FIG. 10 is a photographic representation of a single wire spring device that has been attached to the left ventricle by means of a pair of Dacron tubes.
FIG. 11 schematically illustrates a cardiac girdle type of connecting element. The basic structure of the girdle is depicted in FIG. 11A. The loops formed by the union of pairs of contralateral tabs are shown in FIG. 11B. FIG. 11C illustrates the use of two cardiac girdles attached to the surface of the left ventricular wall.
FIG. 12 illustrates a connecting element of the type known as a cardiac anchor. In FIG. 12A, the anchor is provided with transmural attachment. FIG. 12B shows an alternative form of transmural anchor comprising an internal wall-connecting element and an external wall-connecting element. FIG. 12C illustrates an intramural embodiment of the cardiac anchor.
FIG. 13 depicts an expandable transmural anchor. FIG. 13A shows the basic structure of this anchor, while FIG. 13B illustrates the *in situ* positioning thereof.
FIG. 14 is a photographic representation of a wire spring *in vivo* device that has been attached to the external ventricular wall by means of Dacron tubes.
FIG. 15 is a photographic representation of another wire spring in vivo device that has been attached to the external ventricular wall by means of Dacron tubes.
FIG. 16 illustrates a v-shaped wire spring. The device comprises a series of apices disposed over its entire length, wherein said apices have been further twisted to form loops.
FIG. 17 illustrates another v-shaped wire spring device shown in Fig. 16, wherein said spring comprises regions consisting of the aforementioned v-shaped sections and loops which are interrupted by one or more regions wherein the wire remains substantially straight.
FIG. 18 schematically depicts a hollow element of the connecting element.
FIG. 19 schematically depicts the relative arrangement of the hollow element and the associated four rigid elements of the preferred embodiment of the connecting element shown in Fig. 18.
FIG. 20 depicts a spiral fastener used as part of the connecting element shown in Fig. 18. Fig. 20A provides a general view of the fastener itself, while Fig. 20B illustrates the disposition of four such spiral fasteners within the four rigid elements that are attached to the hollow element of the preferred embodiment of the connecting element shown in Fig. 18.
FIG. 21 is a photographic representation of a wire spring device, in which said device is already attached to three connecting elements of the type depicted in Fig. 18.
FIG. 22 schematically depicts a side view of an exemplary attachment mechanism, showing all three components.
FIG. 23 is a photographic representation of an *in vivo* application of a prototype of the attachment mechanism shown in Fig.20B, in which is shown a Gortex tube attached to the external left ventricular surface using spiral fasteners.
FIG. 24 is a photograph demonstrating an *in vivo* application of a device consisting of a wire spring device, similar to the device illustrated in Fig. 17.
FIG. 25 is another photograph demonstrating an in vivo application of a wire spring device, similar to the device illustrated in Fig. 17.
FIG. 26 is a further photograph demonstrating an another *in vivo* application of a wire spring device, similar to the device illustrated in Fig. 17.
FIG. 27 is a fluoroscopy image of the device shown in Fig. 26 attached to the left ventricle of a sheep.

### DESCRIPTION OF THE PREFERRED EMBODIMENTS

The present invention relates to an *in vivo* device for improving diastolic function of the left or right ventricle of the heart.

It is to be noted that the terms "ventricular", "ventricular surface", "ventricle" and the like are used herein to refer to either the left or right ventricles or to portions thereof. Thus, wherever the description refers to the left ventricle or portions thereof, it is to be appreciated that the teachings derived from said description apply equally to the right ventricle.

A key advantage possessed by all embodiments of the presently claimed *in vivo* device is the fact that said device is capable of exerting elastic forces on the external ventricular wall in a tangential direction, in addition to the externally-directed radial forces. These tangential forces are of importance for the following two reasons:
1. they permit more even distribution of applied forces across the left ventricular wall surface;
2. they assist the diastolic movement of the left ventricle in a manner more similar to its normal physiological movement.

In order to further understand the latter point, it is necessary to further consider the physiological changes in ventricular shape and volume during the cardiac cycle. Thus the normal left ventricle performs a systolic wringing motion with clockwise rotation at the base (of approximately 4.4 degrees) and counterclockwise rotation at the apex (of approximately 6.8 degrees), as seen from the apex (Nagel E, Stuber M, Burkhard B, Fischer SE, Scheidegger MB, Boesiger P, Hess OM: "Cardiac rotation and relaxation in patients with aortic valve stenosis". European Heart Journal 2000;21:582-589). This motion is analogues to the wringing of a wet towel to squeeze the water out; it allows the ventricle to generate high intraventricular pressures, with minimal shortening of the muscle fibers, and thus minimal energy expenditure. It is important to note that the rotation normally occurs during the isovolumic contraction phase, and there is no, or minimal rotation during systolic ejection.

During isovolumic relaxation an untwisting motion is observed, which is directed opposite to systolic rotation, counterclockwise at the base and clockwise at the apex. There is minimal rotation during the filling phase.

Clearly, solely radial expansion of an in vivo device would not provide the optimal assistance in increasing diastolic filling of the left ventricle. The addition of the longitudinal members of the presently-claimed device, however, permits said device to exert tangential forces on the expanding heart, thus assisting the ventricle in its normal untwisting motion, as explained hereinabove.

Referring now to FIG. 1, a main objective of treating a patient with diastolic dysfunction is to cause their abnormal diastolic pressure-volume relation curve (dashed line between 'c' and 'd') to go back to the diastolic pressure-volume relation curve of a normal subject, (dashed line between 'a' and 'b'), by decreasing the diastolic LV pressure for the same LV volume, during the entire diastolic stage of the cardiac cycle, in general, and, by decreasing the end diastolic LV pressure for the same LV volume (indicated by the arrow), in particular. The present invention accomplishes this.

The device of the present invention is based on uniquely applying both a radially outward expansive force or pressure (force per unit area) to the wall region of the left ventricle and a tangentially-directed force or pressure to said wall region, in order to reduce intraluminal hydrostatic pressure of the left ventricle, also known as LV filling pressure, during the ventricular diastolic stage of the cardiac cycle, thereby, improving diastolic function of the left ventricle of the heart, while minimally disturbing systolic function of the heart.

Reduction of hydrostatic pressure within the left ventricle has the beneficial effect of reducing hydrostatic pressure in other cardiac compartments and organs preceding, that is, upstream relative to, the left ventricle in the overall cardiac system, in particular, in the left atrium, and in the pulmonary vasculature of the venous system supplying blood to the atrium. These beneficial effects prevent both dilatation of the atria with propagation to atrial fibrillation, and pulmonary congestion causing symptoms of dyspnea and pulmonary edema.

Normal left ventricular end diastolic pressure (LVEDP) is in the range of about 6 - 12 mm Hg, and the upper end of this range can increase to above 35 mm Hg during conditions of heart failure involving diastolic dysfunction, as a direct result of the left ventricle needing relatively high hydrostatic filling pressures in order to achieve the necessary left ventricular end diastolic volume (LVEDV) for an appropriate cardiac output. Accordingly, an important objective of the present invention is to significantly reduce the hydrostatic pressure in the left ventricle during the diastolic stage of the cardiac cycle, thereby, improving diastolic function of the left ventricle of the heart, while minimally disturbing systolic function of the heart. In particular, fulfilling this objective includes sufficiently reducing left ventricular end diastolic pressure (LVEDP), preferably, down to the normal range of about 6 - 12 mm Hg, during ventricular diastole of the heart.

In addition to the present invention primarily applied for treating subjects having symptoms of diastolic heart failure, by reducing intraluminal hydrostatic pressure (LV filling pressure) of the left ventricle during the ventricular diastolic stage of the cardiac cycle, thereby, improving diastolic function of the left ventricle of the heart, while minimally disturbing systolic function of the heart, the present invention can be used in a variety of other cardiac related and/or non-related monitoring applications, such as pressure measurement applications, and, therapeutic applications, such as in drug delivery applications. For example, the device of the present invention can be used together with an apparatus for time controlled drug delivery or release to the body, in general, and, to the cardiac region, in particular.

It is to be understood that the phraseology and terminology employed herein are for the purpose of description and should not be regarded as limiting. For example, in describing the present invention, the key functionality terms '*elasticity*' and '*resiliency*', and, the corresponding variant terms '*elastic*' and '*resilient*', are considered synonyms, and for the purpose of brevity, while maintaining clarity of description, the terms *'elasticity'* and '*elastic*', are solely used hereinafter, however, it is to be fully understood that the corresponding synonymous terms '*resiliency*' and '*resilient*', respectively, are equally applicable.

The component parts, operation, and implementation of an anatomically compatible and physiologically compatible *in vivo* device for improving diastolic function of the left ventricle of the heart according to the present invention are better understood with reference to the following description and accompanying drawings. Throughout the following description and accompanying drawings, like reference numbers refer to like elements.

The device of the present invention utilizes the physicochemical property and behavior of *elasticity* or *resiliency,* in a relatively simple manner, in appropriately constructed and configured *elastic* or *resilient* components of the device operatively connected to the external surface of a wall region of the left ventricle, for exerting an *elastic* or *resilient* type of the expansive force or pressure to the wall region of the left ventricle, for reducing intracardiac hydrostatic pressure during ventricular diastole of the heart, thereby, improving diastolic function of the left ventricle of the heart, while minimally disturbing systolic function of the heart.

The ventricular device of the present invention may be constructed from either a single type of material, or, from a plurality of different types of materials. Preferably, the ventricular device is constructed from a single type of elastic material, which when appropriately formed, is self-expandable. For example, such material is selected from the group consisting of a pure metal, a metal alloy, and, combinations thereof. Exemplary pure metals are tungsten, platinum, and, titanium. Exemplary metal alloys are nitinol, and, stainless steel.

The ventricular device of the present invention, in general, and, the at least one elastic component, in particular, have dimensions of length, height, and, width, depth, or thickness, each on the order of microns to centimeters, in the range of between about 10 microns to about 8 cm.

The geometry, shape, form, and, dimensions, and, elastic strength, of the ventricular device, in general, and, the at least one elastic component, in particular, are specifically determined, in part, according to the desired or necessary extent or degree of elasticity, for properly and optimally performing the critical function of potentially exerting both radially outward and tangential forces or pressures (in a range of about 5 - 40 mm Hg, preferably, about 10 mm Hg) to the outer wall surface of the left ventricle, in order to properly fulfill the main objective of sufficiently reducing intracardiac hydrostatic pressure during ventricular diastole of the heart, thereby, improving diastolic function of the left ventricle of the heart, while minimally disturbing systolic function of the heart. This includes sufficiently reducing left ventricular end diastolic pressure (LVEDP), preferably, down to the normal range of about 6 - 12 mm Hg, during ventricular diastole of the heart.

Following are description and accompanying drawings for describing and illustrating, respectively, various embodiments of the device of the present invention.

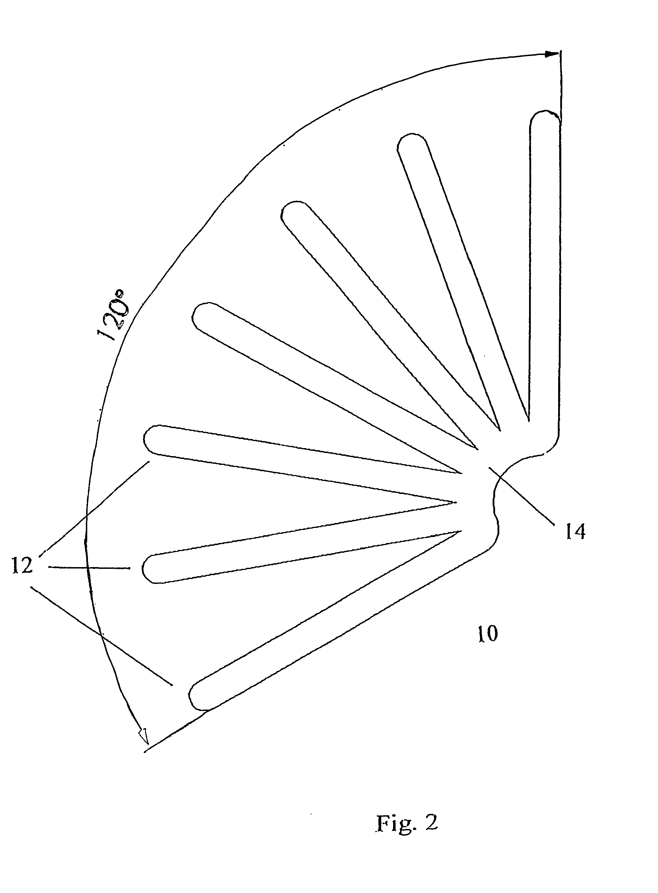

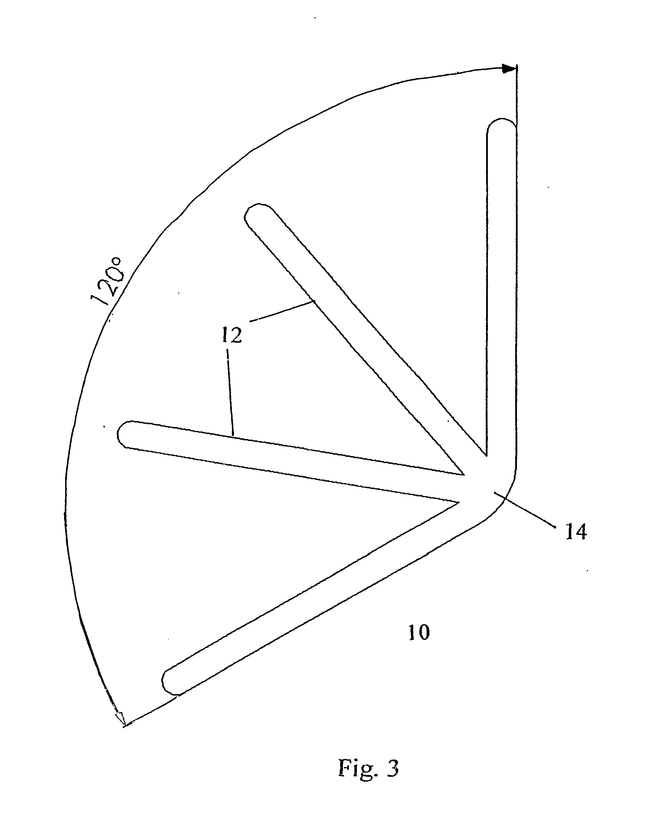

Referring again to the drawings, FIG. 2 depicts one preferred embodiment of the device of the present invention, generally indicated by numeral **10,** comprising a plurality of elongated members **12,** each of which are connected at one of their extremities to curved base element **14.** The device shown in this figure comprises seven elongated members **12,** such that when said device is in the flat, unfolded state (as shown in the view given in FIG. 2), the angle formed between the imaginary longitudinal midlines of the first and last of such members is approximately 120°. This embodiment may be constructed in a variety of sizes in order to accommodate the range of cardiac dimensions normally encountered. In the particular embodiment depicted in FIG. 2, each elongated member **12** is of length 70 mm, including the width of base member **14,** with which said elongated member is continuous. Another example not part of the invention is depicted in its unfolded condition, in plan view, in FIG. 3. In this case, the device comprises four elongated members **12,** the angle formed between the imaginary longitudinal midlines of the first and last of such members being approximately 120°C. In devices of the type depicted in FIGS. 2 and 3, the elongated members **12** may be constructed such that they have any suitable width. In the examples of such devices shown in FIGS. 2 and 3, said elongated members have a width of 6 mm. It is to be noted that the longitudinal elongated members may take many different forms, in addition to those depicted in FIGS. 2 and 3. For example, said members may be perforated by a series of small holes in order to allow attachment thereof to the ventricular wall. In addition, the members may (in plan view) be curved or otherwise formed into non-linear shapes. Furthermore, within one device, different members may be constructed in a plurality of shapes and sizes.

The embodiments of the device depicted in FIGS. 2 and 3 may be constructed of any suitable elastic material. Preferably, said embodiments are constructed of metal wires or tubes. Examples of metals possessing the required physical properties include (but are not limited to) stainless steel 316 and NITINOL (Nickel Titanium), both of which are biocompatible metals that are commercially available in the form of wires or tubes. For examples, wires of both materials may be obtained from Allvac Inc., Monroe, NC.

In the case of wires, industrial bending machinery may be used to bend the wire into the desired shape.

In the case of tubes a "cut out" method may be used. In this type of method, selected areas of the metal of the tube are removed, for example, by laser cutting, until only the desired geometry, shape, and dimensions, remain.

Exemplary dimensions of the embodiments of the device depicted in FIG. 2 are as follows: Longitudinal length, (that is, the length extending along imaginary central longitudinal axis, from the top of elongated members **12** to the lower surface of the base element **14** of the device) is in the range of between about 0.5 cm to about 10.0 cm, preferably, about 6 cm. The diameter across the widest end, that is, the distance spanning across the top or free ends of the elastic arms or extensions, is in the range of between about 0.1 cm and about 6.0 cm, preferably, about 3 cm. The angle between any two of the longitudinal elastic arms or extensions can be in the range of 5 - 180 degrees, preferably about 30 degrees. The average depth or thickness of the metal is in the range of between about 0.01 mm (10 microns) to about 5.0 mm (5000 microns), preferably, about 0.3 mm (300 microns).

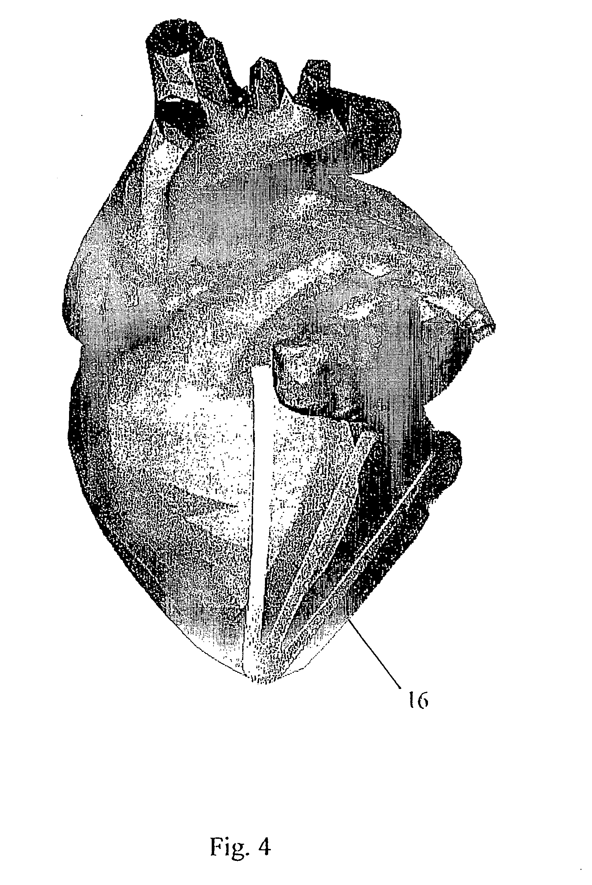

FIG. 4 depicts the device described hereinabove (and shown in FIG. 3) in its *in situ* position on the external surface of the left ventricle **16.** The device may be connected or attached to the external surface of the heart by the use of any suitable conventional material or means, including (but not restricted to) biocompatible pins, biocompatible needles, biocompatible spikes, biocompatible screws, biocompatible clamps, biocompatible glue, biocompatible adhesion, surgical sutures, and, combinations thereof, having dimensions of length, height, and, width, depth, or thickness, each on the order of microns to centimeters, in the range of between about 10 microns to about 8 cm. In addition, the present invention also provides certain novel connecting elements that may be used to attach the above-described device to the external surface of the heart in a manner such that said device is held in close apposition to said external surface, thus resulting in maximal transduction of the potential energy of the elastic component into the expansive kinetic energy used to assist in diastolic filling of the left ventricle.

One preferable embodiment of such connecting means includes the use of a plurality of open-ended tubes constructed of a biocompatible material, said tubes being connected to the external surface of the heart by means of surgical sutures or suture clips or any other suitable conventional means, such that said tubes are disposed in an essentially longitudinal orientation. Tubes of any suitable biocompatible material may be used; preferred materials include Dacron and polytetrafluorethylene (PTFE). Preferably, the tubes have an internal diameter in the range of 0.2 - 1.4 cm and a length in the range of 1 - 5 cm. Suitable Dacron tubes originally intended for use as arterial grafts are highly suitable for this purpose, and may be commercially obtained from C. R. Bard, Inc., Murray Hill, NJ, USA.

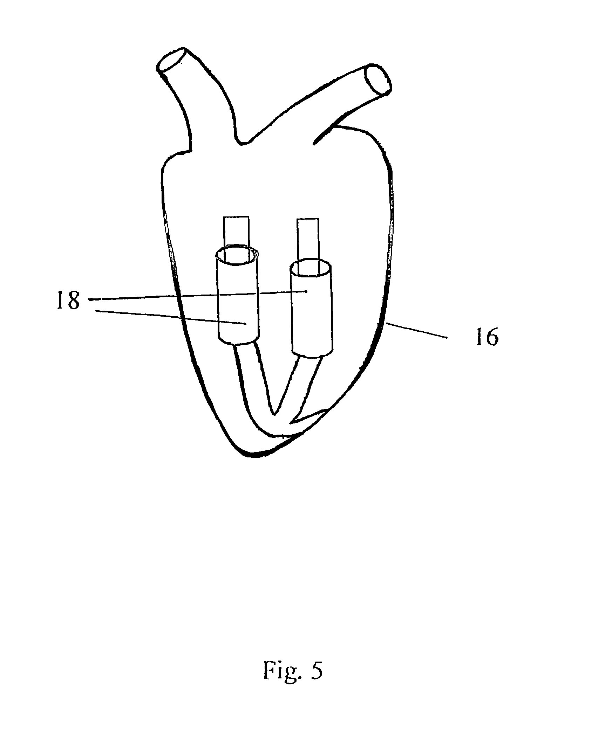

FIG. 5 illustrates an example of the above-described device that has been attached to the external surface of the left ventricle **16** by means of Dacron tubes **18.** Supplementary connecting means (not shown), selected from the group of means and materials given hereinabove (e.g. sutures, glue, pins etc.) are additionally used at discrete points along the device in order to provide extra stabilization of said device in relation to the ventricular surface.

The embodiments of the device of the invention described hereinabove and depicted in FIG. 2 may be inserted in place using a minimally invasive surgical procedure, such as a thoracoscopy, or, thoracotomy, with a relatively small diameter delivery system for delivering and deploying the ventricular device into the body, in general, and to a left ventricular cardiac outer wall surface, in particular.

Preferably, base element **14** is self-expanding, in order to facilitate the use of minimally invasive insertion procedures such as those described above.

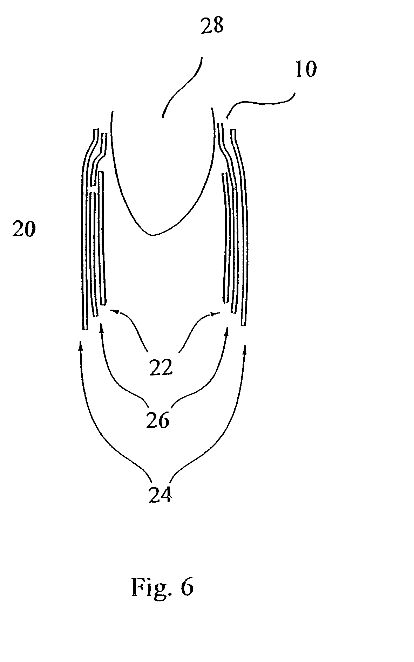

Techniques and equipment of thoracoscopy deployment are well taught about in the prior art, however, for enabling implementation of the method and device of the present invention, an example is provided herein. FIG. 6 is a schematic diagram illustrating an example of a thoracoscopic delivery system **20** for delivering the device to its attachment site on the external cardiac wall. Delivery system **20** is basically structured as a three layered sleeve. The device of the invention **10** is supported between an inner support sleeve **22,** an outer support sleeve **24,** and, an axial support sleeve **26,** which prevents said device **10** from slipping backwards. Delivery system **20** is maneuvered and placed in the body so that it envelops the heart **28,** and, after maneuvering and positioning the device **10** in place, inner support sleeve **22** is gradually retracted out, while outer support sleeve **24** is used to compress the device **10** towards the ventricular outer wall surface. At this stage, the device **10** is connected to the outer wall surface of the left ventricle, followed by withdrawing outer support sleeve **24** and axial support sleeve **26.**

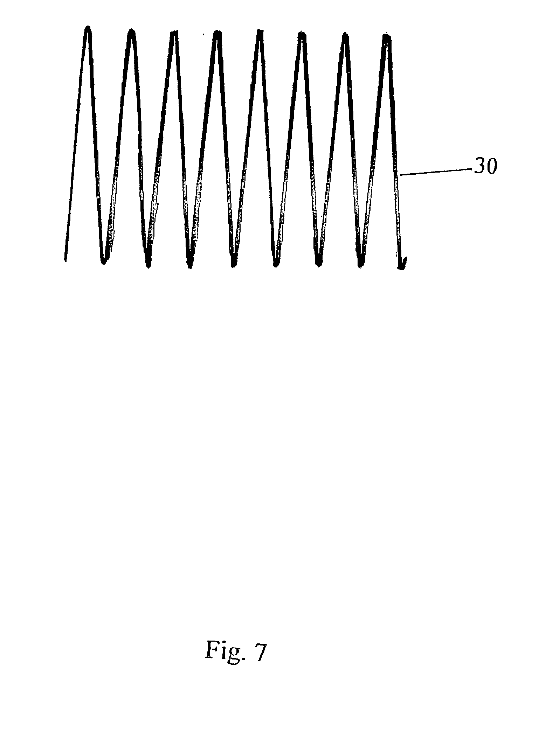

In an example not part of the invention, the elastic component comprises a wire spring **30,** wherein said spring is formed such that it contains along its length one or more angled portions, each of said angled portions being approximately v-shaped or u-shaped, as indicated in FIG. 7. One consequence of using this shape of wire spring as the elastic component is that said elastic component is able to exert tangential as well as radial forces on the external surface of the heart to which it is attached. Thus, the radial forces arise from the bending of the whole spring when it is connected to the surface of the heart, and its envelope shape adapted to the curvature thereof. The tangential forces, on the other hand, are a function of the v-shaped or u-shaped bends of the wire spring.

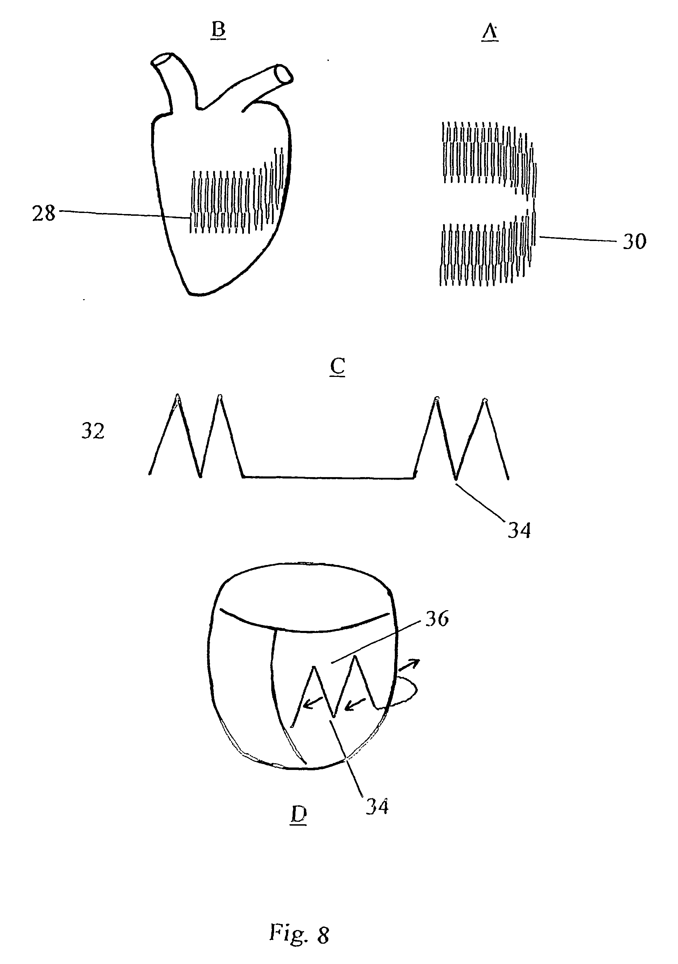

FIG. 8A depicts one example of this wherein the wire spring **30** is formed into a series of v-shaped bends in an uninterrupted manner over its entire length.

FIG. 8B shows the same spring *in situ* extending over most of the width of the left ventricle of the heart **28.**

FIG. 8C illustrates another wire spring type of elastic element for use in accordance with the present invention. It will be noted that in this case, the spring **32** comprises two laterally-placed v-shaped angled sections **34** separated by a linear section.

As indicated in FIG. 8D, this particular example is designed such that when attached to the heart, the angled sections **34** are situated over the lateral portions of the external surface of the left ventricular wall **36.** The arrows in this figure indicate the position and direction of the tangentially-directed forces exerted by the angled v-shaped portions of the spring on the left ventricular wall **36.**

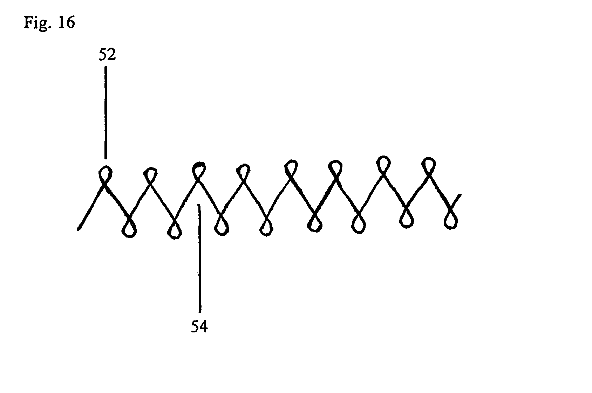

FIG. 16 depicts an alternative form of the wire-spring elastic element generally indicated as **54,** wherein the apex of each v-shaped section is further twisted around the longitudinal axis of said, such that it forms an essentially circular loop **52.** In this example, there is one loop **52** for each v-shaped section, but in other examples there may be two or more such loops, as may be found to be most suitable for assisting diastolic function of the left or right ventricle.

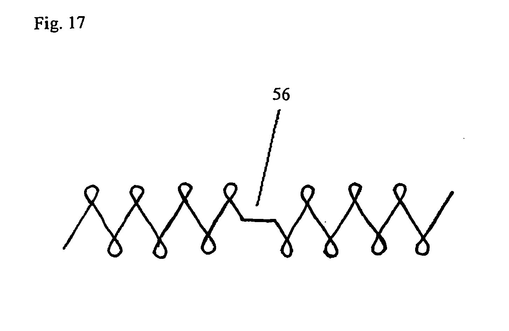

In the example depicted in FIG. 17, however, the aforementioned wire-spring elastic element comprises regions consisting of the aforementioned v-shaped sections and loops alternating with adjacent regions wherein the wire remains substantially straight **56.** In this example there is one substantially straight section **56,** but in other examples there may be two or more such substantially straight sections, as may be found to be most suitable for assisting diastolic function of the left or right ventricle.

While the inventors do not wish to be bound by a particular hypothesis or any other theoretical considerations, it is to be understood that the aforementioned essentially circular loops 52 function in the following manner: during every contraction of the ventricle the device is constricted, causing each loop to expand (against its basal state), thus causing a force directed towards reversing the contraction, and expanding the ventricle. The sum of these forces is a normally-acting force, or in other words a radially-outward, expansive force, which assists in the filling or expansion of the ventricle during the diastolic phase of the cardiac cycle. This allows filling of the ventricle with lower filling pressures, thus assisting the diastolic function of the ventricle to which said device is attached.

Thus, any of the wire spring devices may, for example, be surgically connected to the external surface of the left or right ventricle, preferably by means of one or more specialized connection elements all of which will be described in more detail hereinbelow. During systolic contraction of the heart muscle, the wire spring will be placed in a compressed state, absorbing potential energy, which in turn will be transformed into kinetic energy during the diastolic phase, thereby assisting in the filling of the left ventricle. This will significantly reduce the hydrostatic pressure in the left ventricle during the diastolic phase of the cardiac cycle, thereby, improving diastolic function of the left ventricle of the heart, while minimally disturbing systolic function of the heart. In particular, fulfilling this objective includes sufficiently reducing left ventricular end diastolic pressure (LVEDP), preferably, down to the normal range of about 6 - 12 mm Hg, during ventricular diastole of the heart.

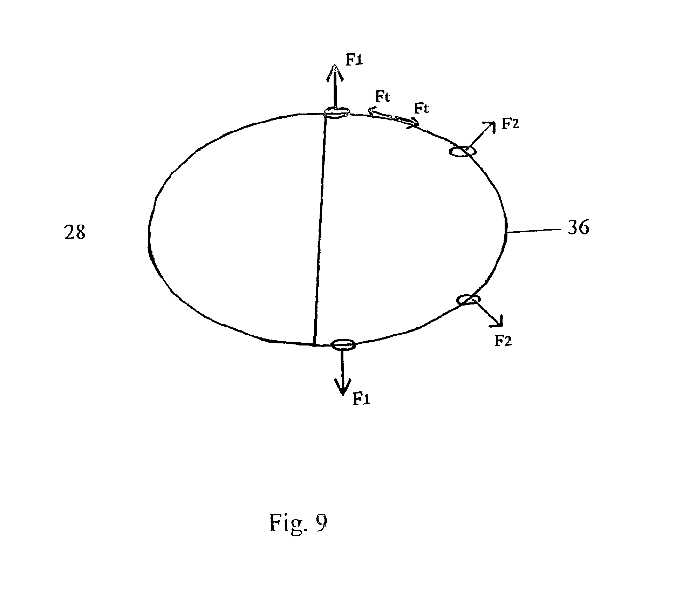

FIG. 9 is an illustrative plan view of the heart **28** showing the direction of the forces exerted by the device illustrated in FIG. 8D on the external surface of the left ventricular wall **36.** The arrows labeled as **F1** indicate the direction of the radial forces acting on the attachment points of the device to the ventricular wall **36** (shown as flattened ellipses). The arrows labeled as **Ft** indicate the tangentially-directed forces, while the arrows **F2** indicate the direction of the vector sum of the various forces acting on the attachment points. It will be seen from this figure that said vector sum direction is in a direction that will lead to an outward expansive (i.e. inflating) movement of the left ventricular wall.

As mentioned hereinabove, the presently-discussed device may be connected or attached to the external surface of the heart by the use of any suitable conventional material or means, including (but not restricted to) biocompatible pins, biocompatible needles, biocompatible spikes, biocompatible screws, biocompatible clamps, biocompatible glue, biocompatible adhesion, surgical sutures, and, combinations thereof, having dimensions of length, height, and, width, depth, or thickness, each on the order of microns to centimeters, in the range of between about 10 microns to about 8 cm. In addition novel connecting elements may be used to attach the above-described device to the external surface of the heart in a manner such that said device is held in close apposition to said external surface, thus resulting in maximal transduction of the potential energy of the elastic component into the expansive kinetic energy used to assist in diastolic filling of the left ventricle.

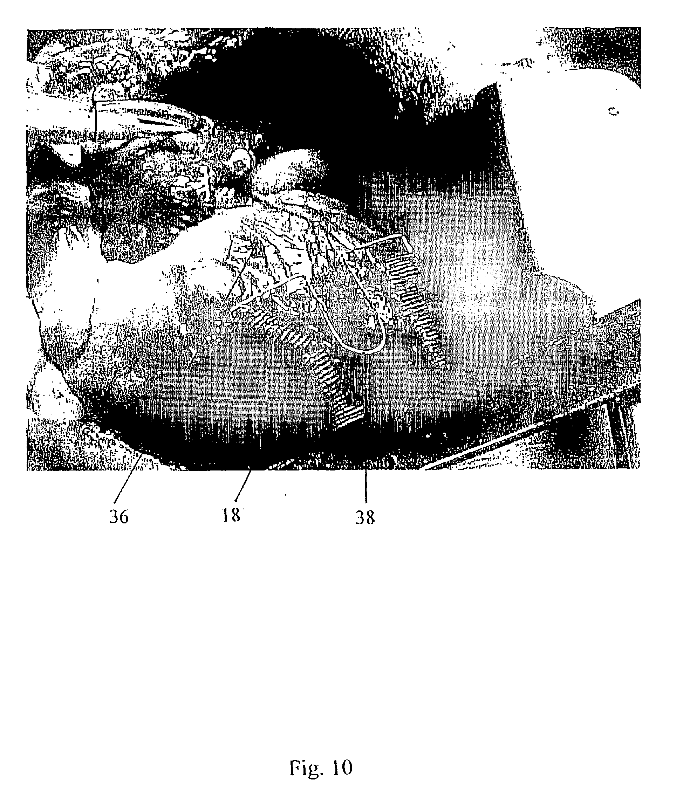

One preferable type of connecting means for use with the wire spring device involves the use of one or more open-ended Dacron or polytetrafluorethylene (PTFE) tubes (as described hereinabove), said tubes being connected to the external surface of the heart by any suitable means including, but not limited to, surgical sutures and suture clips. The tubes are disposed in an essentially longitudinal orientation, such that vertically-orientated end sections of the wire spring can be inserted therein. Preferably, the tubes have an internal diameter in the range of 0.2 - 1.4 cm and a length in the range of 1 - 5 cm. FIG. 10 is a photographic representation of a single wire spring device containing a single, medially placed u-shaped angled section, **38** inserted into two Dacron tubes **18** that have been sutured to the external surface of the left ventricular wall **36.**

Metal wires used for constructing this device include (but are not limited to) stainless steel 316 and NITINOL (Nickel Titanium) wires, both of which are biocompatible and are readily available from commercial suppliers (e.g. Allvac Inc., Monroe, NC). Preferably, wires having diameters in the range of 0.1mm to 2mm are used in the construction of the wire spring device.

The presently-discussed device may be manufactured by taking a 10 - 40 cm length of metal wire and bending it into the desired shape (e.g. as depicted in FIGS. 8A - 8D) by means of a wire bending jig, a hand bending with pliers and vice or industrial wire bending equipment. For example, in the case of hand bending, the exact desired configuration may be first marked on millimetric paper, after which the wire may be manually bended using pliers at one end of the wire and an instrument to stabilize the second end of the wire. For large scale production, industrial wire bending equipment may be used.

In another mechanism, said mechanism comprises the following three main components:

### 1. The hollow element.

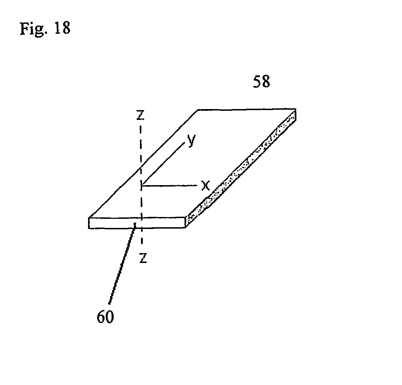

The device which is to be attached, or a part of it, is inserted into the internal space of the hollow element. The hollow space allows movement of the inserted device in a plane composed of X and Y axes, which are lateral axes, but does not allow free movement on the Z axis. This permits a device that is attached to the external surface of a ventricle of the heart to be used in situations where it is required to apply only Normal forces to the ventricular surface (forces in the Z direction, as shown in FIG. 18). This may be beneficial since during normal ventricular contraction there is a twisting motion of the myocardium, and said twisting motion would cause a significant, mechanically undesirable strain on the attachment areas if lateral motion (in the X and Y axes) was not free. This fact permits the device to move freely in the lateral axes, but to apply a force on the ventricular surface when moving in the Normal axis (Z in FIG. 18), thus concentrating the effects of the device in that direction and reducing undesirable lateral stress on the attachment area. Stress on the attachment area is to be avoided, since it may cause an increase in myocardial oxygen requirements, may cause ischemia, and may even cause rupture of the myocardium in the attachment area. FIG. 18 illustrates an exemplary hollow element 58, as described above. In this figure, the hollow space 60 within the connecting element is bounded by closed sides, as indicated by the gray filling. The axes of movement are illustrated to emphasize the fact that a part of a device that is inserted into the hollow area 60 can move freely in the X and Y axes, without applying a force on the hollow element, but any movement in the Z axis will apply a force in that direction on the hollow element and on anything attached to it, such as the external ventricular surface.

Exemplary materials for the hollow element are biocompatible fabrics, biocompatible mesh, biocompatible plastics and biocompatible metals. Particularly preferred materials are Gortex and Dacron. While the hollow element may be manufactured in any suitable form, preferably said hollow element is a Gortex tube, similar to the tubes used as arterial grafts.

### 2. The rigid element.

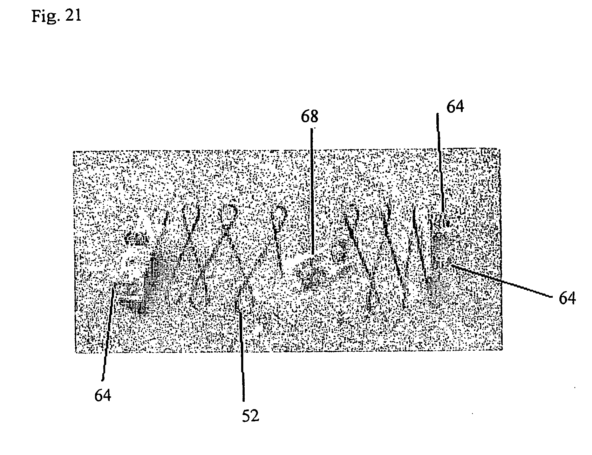

The rigid element serves three important purposes:
1) It creates a predetermined course for the fastener (the fastener will be further described in the next paragraph as the third element of the attachment mechanism). The predetermined course is important especially if the fastener is a spiral or helical fastener, which would otherwise be difficult to insert into the hollow element which may be soft, for example, a fabric. The predetermined course allows easy insertion of the fastener into the hollow element and through it, into the tissue to which the device will be attached (for example, the external surface of a ventricle of the heart). This is essentially a helical track formed in the rigid element.
2) It prevents movement of the hollow element during insertion of the device. This might occur, for example, when inserting a spiral fastener into a Gortex tube. If the spiral fastener would be inserted directly into the Gortex, in a corkscrew like manner, then during the helical insertion the Gortex tube itself, which is flexible, would be caused to rotate, due to the lateral forces of the helical insertion. However, if the same helical fastener is inserted into a rigid element within the same Gortex tube, then during the insertion the tube itself is not disturbed. Furthermore, the helical fastener can be pre-inserted into the rigid element so that the tip is already inside the rigid element, going all the way through (thus creating a pre-determined course for the fastener), but without the tip penetrating to the other side.
3) It allows the tube (hollow element) and the fastener to be supplied by the manufacturer as one unit, so that the attachment mechanism is approximated onto the organ (e.g. cardiac ventricle) to which it will be attached, and then quickly and easily attached thereto. An example of this is the wire-spring elastic component disclosed and described hereinabove. Thus, the wire spring can arrive from the manufacturer with several attachment mechanisms already attached thereto, as indicated by part number **68** in FIG. 21. Furthermore, the elastic device-attachment mechanism assembly may also be supplied with the helical fasteners fully or partially pre-inserted through the rigid element, thus permitting the surgeon to approximate the device to the external ventricular surface in order to determine correct placement. The helical fasteners may then be quickly and easily inserted into the ventricular wall in a corkscrew like manner.

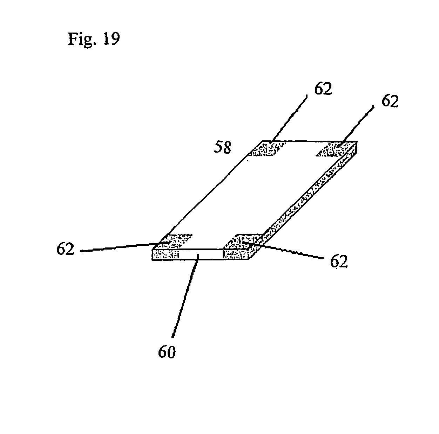

FIG. 19 illustrates the hollow element **58,** the opening **60** of the element, and four rigid elements **62.** In this example the rigid elements are cubical in shape, however this is only an example, and any other shape appropriate for inserting the fasteners may be used. Additionally, there may be one or more such rigid elements. The rigid elements may be evenly or unevenly dispersed on the hollow element, and they may be on one or both sides of the hollow element.
Exemplary materials for the rigid element are biocompatible plastics and biocompatible metals.

One example of a preferred material is silicon. The attachment mechanism may comprise one or more silicon extrusions (the rigid elements) embedded into a Gortex tube (the hollow element)of the type commonly used as arterial grafts.

### 3. The Fastener

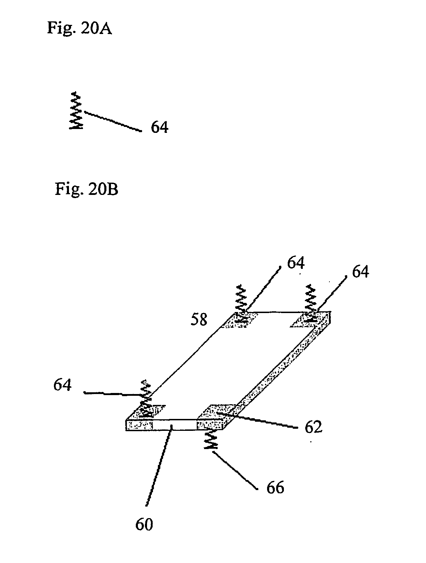

The fastener is the element that adheres, attaches or fastens the complete attachment mechanism (with the device inserted into the hollow element) onto the target tissue or organ area. A preferred example of such a fastener, depicted in FIG. 20A, is a helical or spiral screw-thread fastener **64.** Other types of fasteners may be similarly used, including nails, screws or anchors. The helical fastener may be inserted in a corkscrew like manner into the attachment surface. The fastener is carefully designed to ensure that the spiral cannot be pushed too far into the attachment surface (e.g. the external ventricular surface).

The spiral tip may have a sharp point at its distal end, and further may have a spiral tip at its proximal end. Twisting the hub causes the spiral tip to be drawn into the attachment surface (ventricular surface). The hub abuts the surface when the spiral tip has been fully inserted, thereby preventing the fastener from being pushed too deeply under the surface, and avoiding any possible tissue injury.

The fasteners may be applied singly, typically in spaced-apart patterns on the hollow element. Exemplary materials for the fastener include biocompatible metals and biocompatible plastics. Particularly preferred materials include stainless steel and Nitinol.

Preferably the attachment mechanism comprises a stainless steel spiral fastener inserted into a Silicon extrusion (rigid element) which is in turn embedded into a Gortex tube (hollow element) of the type employed as arterial grafts.

FIG. 20B illustrates an exemplary attachment mechanism comprising a hollow element **58** (possessing an opening to the interior cavity **60**) into which is inserted four rigid elements **62.** Spiral fasteners shown as numerals **64** are shown to be in a fully inserted state within the rigid elements. In this situation said fasteners **64** are actually already inserted into the attachment surface (for example into the external ventricular surface), which is not shown in the figure. The fastener indicated by part number **66** is only partially inserted into the rigid element, illustrating the manner in which the device may optionally be supplied by the manufacturer (i.e. with partially inserted fasteners), and further illustrates the manner in which the attachment mechanism can be approximated to the ventricular surface, and then fastened to it in a corkscrew like manner.

The attachment mechanism may essentially be a ring on the end of a spiral fastener. The device to be attached is inserted into the ring.

FIG. 21 is a photographic representation of a stainless steel wire spring device, similar to the device illustrated in FIG. 17, comprising a series of superior and inferior apices, each of which is twisted around its longitudinal axis so as to form a loop **52.** Three attachment mechanisms 68 each having hollow elements **58** manufactured from Gortex tubes are also shown. Two attachment mechanisms are on the two lateral sides of the wire spring device and a third is in the middle area of the wire spring, covering the substantially straight section. While the rigid elements within the Gortex tubes are not seen in this figure, two spiral fasteners **64** are clearly visible protruding from one face of each of the hollow elements **58.** The combination of wire-spring device and attachment mechanism depicted in FIG. 21 may be prepared such that it can be obtained pre-sterilized from the manufacturer, in a form that is ready for attachment to the external ventricular surface by a surgeon. The attachment process is quick and safe: the device is approximated to the left ventricular surface, the appropriate placement area is decided, and then the surgeon attaches the device to the ventricle by inserting the spiral fasteners in a corkscrew like manner into the ventricular tissue. *In vivo* studies with this design performed by the inventors did not reveal any adverse effects associated with this insertion process.

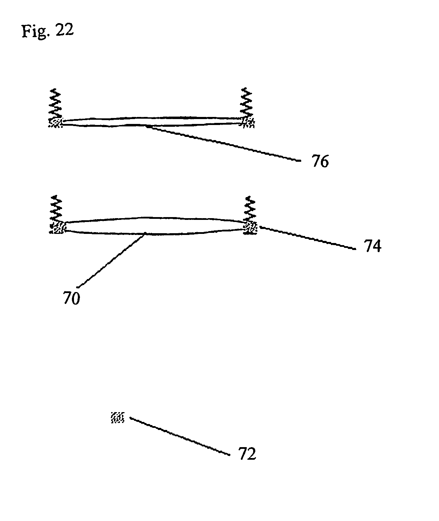

FIG. 22 illustrates a side view of an exemplary attachment mechanism, showing all three of the key components that were defined and described hereinabove. The hollow element, which is indicated by part number **70** before completing the attachment process, becomes constricted **76** after the attachment is complete. The rigid elements **72,** into which the spiral fasteners **74** are inserted, are shown in this figure as being located on two sides of the hollow element.

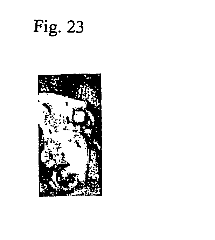

FIG. 23 is a photographic representation of an *in vivo* application of the attachment mechanism that was depicted in FIG. 20B. A Gortex tube (as the hollow element) is shown to be attached to the external left ventricular surface by means of stainless steel spiral fasteners. The spiral fasteners are inserted into a rigid element constructed of silicon (not shown in the figure) inside the Gortex tube. The Gortex tube is fully inserted into the left ventricular wall, and only the end of the spiral fastener, which holds the Gortex tube in place is visible, since the body of the spiral fastener has already engaged the tissue of the left ventricular wall.

In this example, approximately two rotations fully engaged the spiral fastener inside the ventricular surface, leaving the hub pressed against the Gortex hollow element. In other examples the spiral fastener may be designed such that one rotation, or more than two rotations will be needed in order to fully engage the fastener.

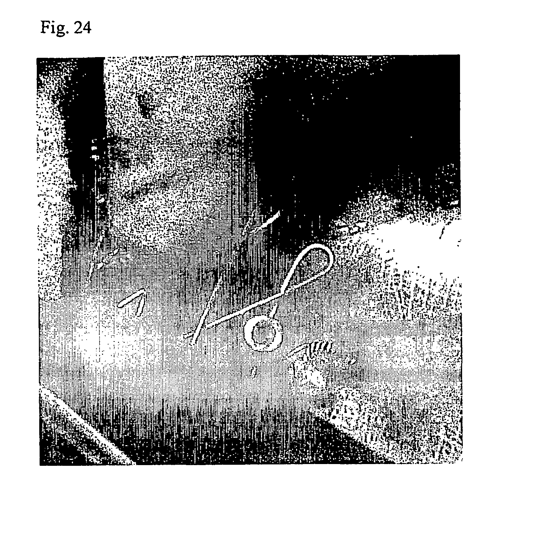

FIG. 24 is a photograph demonstrating an in *vivo* application of a stainless steel wire spring device, similar to the device illustrated in FIG. 17. A spiral fastener, held by forceps, is clearly visible in this figure.

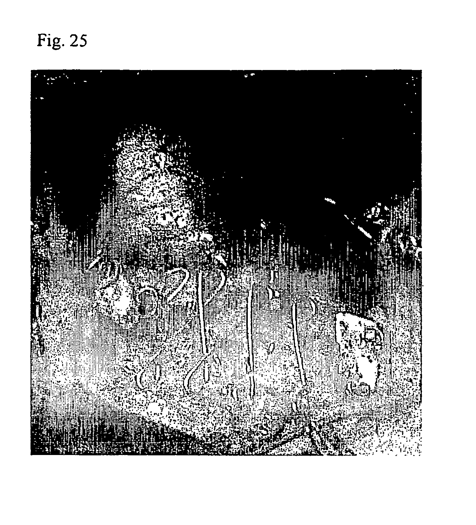

FIG. 25 is a photographic representation of an in vivo application of a wire spring device, similar to the device illustrated in FIG. 17. The device is attached to the external surface of the left ventricle of a sheep. The attachment is achieved by three Gortex tubes, each applied to the ventricular surface by two spiral fasteners; only two such Gortex tubes are visible in the photograph. In the inventor's in vivo studies performed with this design, no adverse effects were reported during the process of insertion, or during three month' follow-up.

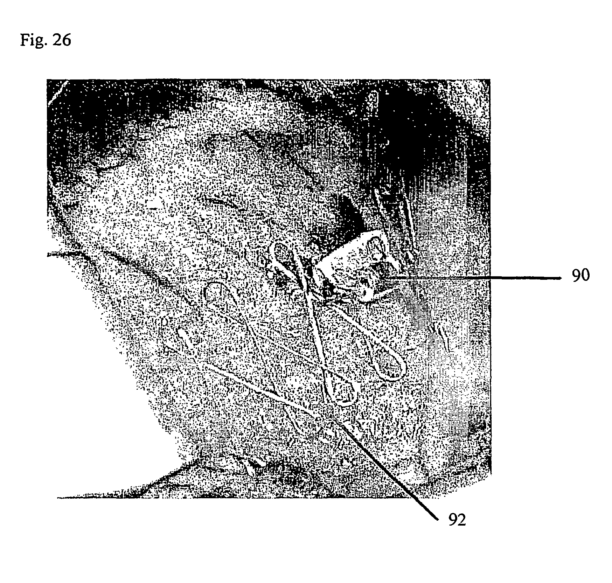

FIG. 26 is a photograph demonstrating an in vivo application of a wire spring device, similar to the device illustrated in FIG. 17. The device is attached to the external surface of the left ventricle of a sheep. The attachment is achieved by three Gortex tubes, each applied to the ventricular surface by two spiral fasteners. Only one such Gortex tube is visible in the photograph, in this case the middle Gortex tube which covers a substantially straight middle region of the device. An optional element of the device - an elongation enabler, or preload enabler - is indicated by part number **90.** In this example, the wire spring of the device is not one single wire, but is composed of two different wire springs that are held together by a cylinder connecting the two middle ends of the wires in the substantially straight area of the device. This is beneficial since it enables a single device to create varying forces, relating to the length of the wires. If an increased force is required the device may be elongated - thus increasing the preload. If a reduced force is required the device may be shortened - thus reducing the preload. This enables one device to be adaptable for different patients requiring different assistance forces, due to different cardiac sizes or to different severity of disease. A further optional preload determining element **92** is also visible in this figure. In this example, it is simply a surgical suture that connects between adjacent loops of the device. This allows constriction of the device before and during its attachment to the ventricular surface. Following attachment of the device to the ventricular surface the sutures may be cut, thus releasing the full force capacity of the device and allowing it to fully expand to the resting state, thus applying an expansion force on the ventricle, thereby assisting diastolic function. Other types of preload determining elements are possible, such as a comb-like mechanism in which the comb arms are inserted into the loops of the device in order to constrict it before its attachment to the ventricular surface.

In the inventors' *in vivo* studies performed with the device depicted in FIG. 26, no adverse effects were observed either during insertion, or following the cutting of the surgical sutures thereby allowing the device to fully expand. In addition, no adverse effects occurred during alteration of the preload of the device by means of elongating and shortening the wire spring.

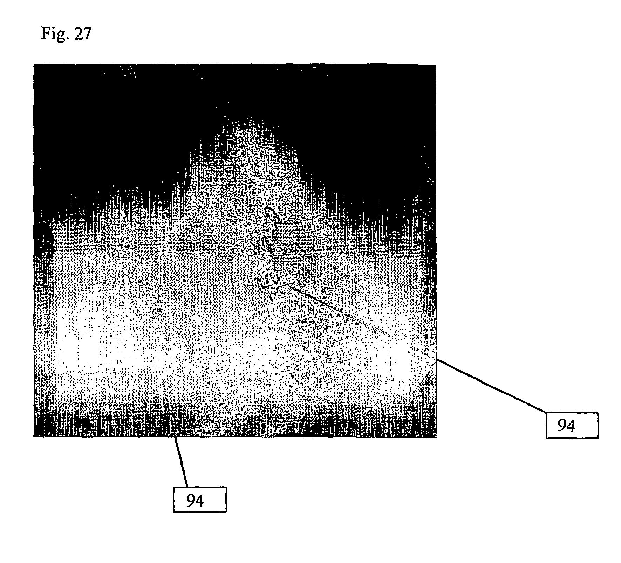

FIG. 27 is a fluoroscopy image of the device shown in FIG. 26, attached to the left ventricle of a sheep. The fluoroscopy image was acquired after closure of the chest, with the device attached to the beating left ventricle of the sheep's heart. The spiral fasteners **94** attaching the wire spring device to the ventricular surface are clearly seen. (Not shown are the Gortex tubes and the Silicon elements within them, into which the spiral fasteners are inserted). No adverse effects were reported during or after the study, and the sheep was returned in good clinical condition to the farm, with the device still attached to the left ventricle.

In addition to the connecting means described hereinabove, the use of several other types of connecting elements which may be used for connecting the various embodiments of the presently-claimed *in vivo* device to the external ventricular wall is also described.

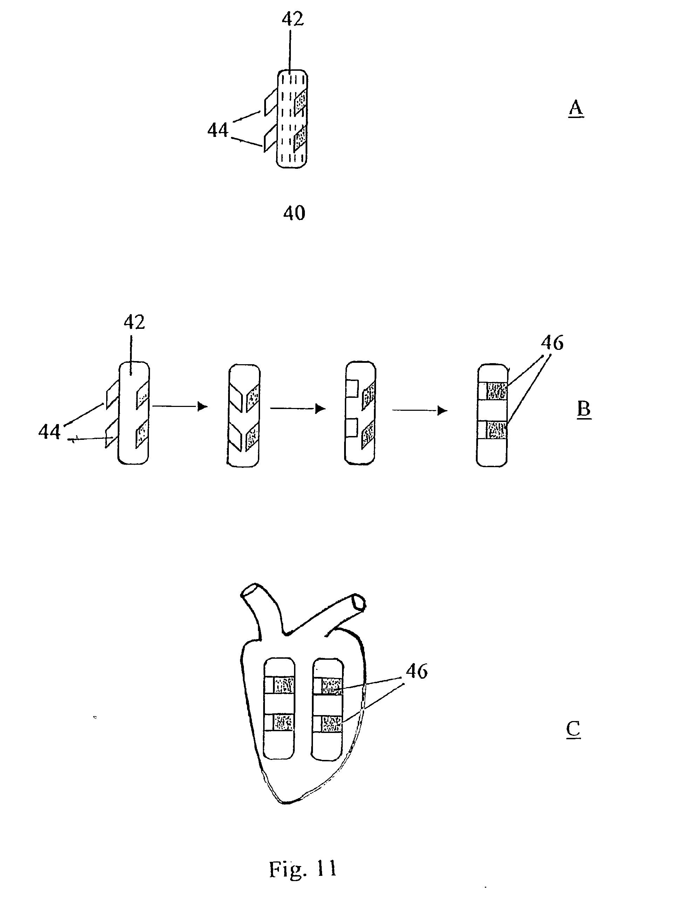

One such type of connecting element is the cardiac girdle depicted in FIG. 11. As shown in FIG. 11A, the cardiac girdle **40** comprises a thin patch **42** which may be attached to the external surface of the ventricular wall. Extending laterally from said patch is a plurality of tabs or straps **44,** arranged in contralateral pairs. In a preferred example, the cardiac girdle comprises two such contralateral pairs of tabs. The length of each tab is such that it can overlap with its contralateral partner, thus forming a loop **46,** as shown in FIG. 11B. The thin patch element of the cardiac girdle is adhered to the external LV wall by means of suturing, gluing or pinning, and the loops formed by each contralateral pair of tabs are used to grip portions of the in vivo device. The patch may be orientated on the external ventricular wall such that said loops, once formed, are arranged in either an essentially horizontal or an essentially vertical orientation. The cardiac girdle may thus be used to grip either horizontally-disposed or vertically-disposed members of the in vivo device. FIG. 11C illustrates the use of two cardiac girdles which have been attached to the external surface of the left ventricular wall such that the loops 46 are orientated horizontally, thus allowing each of said girdles to grip a vertically-disposed longitudinal member of a device of the invention.

The cardiac girdle may be made from any suitable biocompatible material. Examples of such materials include Dacron and polytetrafluorethylene (PTFE), both of which possess the required mechanical strength in order to function as connecting means, and which may be woven into meshes.

A cardiac girdle, as described above, may be inserted into the thoracic cavity and used to connect an *in vivo* device to the external ventricular wall in the following manner:

The heart is surgically exposed following midline sternotomy and pericardiotomy. The heart is then measured in various dimensions (apex to base, circumference at base and midway between base and apex) in order to assist with selection of an *in vivo* device and cardiac girdle of an appropriate size. The girdle may then be attached to the external ventricular wall by means of pinning, gluing or suturing. In the latter case, the cardiac girdle is sutured to the myocardium using multiple partial-thickness (deep) interrupted stitches, taking care not to compromise any of the epicardial coronary arteries. When pinning is used, the fabric may be attached to the myocardium using e.g. multiple star-like, splitting non-retractable tacking pins, avoiding the epicardial coronary arteries. The in vivo device, constricted temporarily to the heart size by means of a constriction mechanism, is now positioned on the external surface of the heart and locked within the girdle by means of closure of the aforementioned tabs or straps, to form retaining loops. The constriction mechanism is removed from the device to allow the device to exert expansive and tangential forces on the external ventricular wall. Following attachment of the girdle and device, the heart is observed in order to ascertain that detachment of the fabric patch of the girdle from the myocardium has not occurred at any point. Final fixation of the device within the girdle is now performed using interrupted stitches.

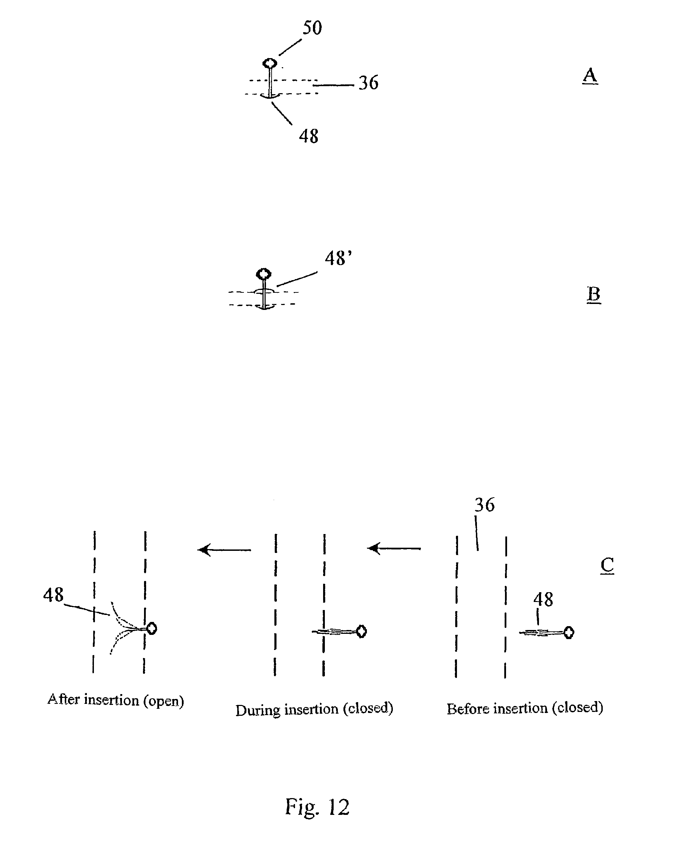

Another type of connecting element is the cardiac anchor, three examples of which are illustrated in FIG. 12. Each anchor is composed of the following two main elements:
1. *A wall-connecting element* **48** for attachment of the anchor to the left ventricular wall. This element may be connected to said ventricular wall by a transmural or an intramural attachment mechanism. In addition, the wall-connecting element may also be attached to the ventricular wall by means of biocompatible glue, pins, hooks, sutures or any other convenient means.
2. *A device-connecting element* **50** for attachment of an *in vivo* device to the anchor. This element may take one of several different forms including, for example, a ring, into which the device is attached or sutured. It may also incorporate a locking mechanism. In addition, biocompatible glue, pins, hooks or sutures etc. may also be used for attaching the anchor to the *in vivo* device.

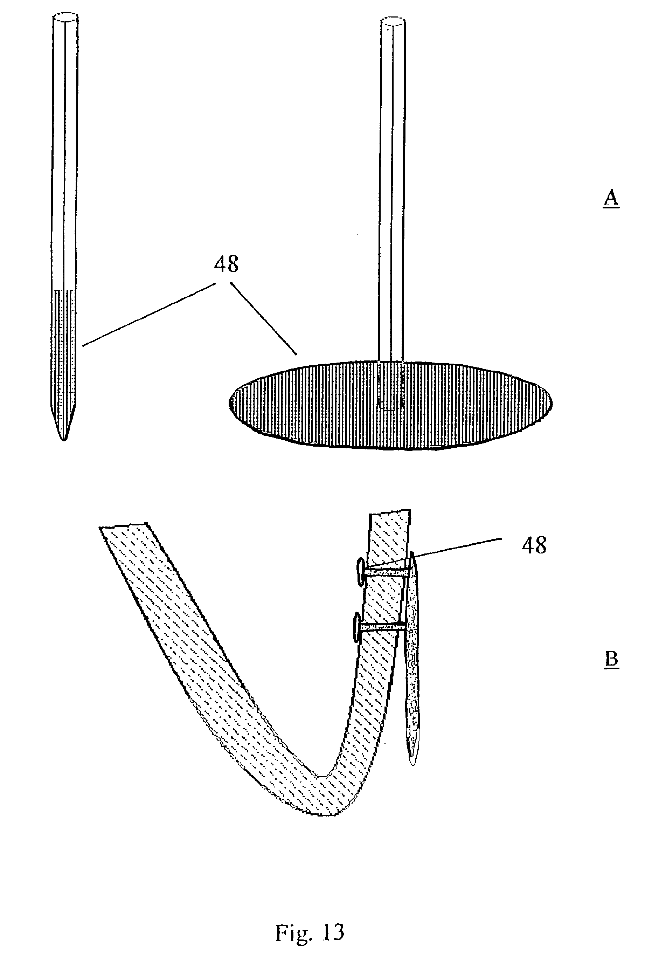

As illustrated in FIG. 12A, the anchor is provided with transmural attachment, wherein the wall-connecting element **48** is located on the inner side of the left ventricular wall **36** (i.e. within the ventricular cavity), while the device-connecting element **50** is situated external to said ventricular wall. Preferably, this anchor is provided in the form of an expandable transmural anchor, as shown in FIG. 13A. In this case, the anchor is inserted into the left ventricular wall while in its closed, or retracted, condition. Once the wall-connecting element **48** of said anchor has passed through the ventricular wall and becomes located within the ventricular cavity, said wall-connecting element is opened, by means of, for example small spring elements, thus fixing the anchor to the ventricular wall **36,** as shown in FIG. 13B.

In another example (FIG. 12B), the transmural anchor is further provided with a second wall-connecting element **48'** which, after insertion of said anchor into the ventricular wall **36,** becomes situated on the external surface of said wall. In this way, the anchor is stabilized by virtue of the fact that the ventricular wall becomes "sandwiched" between the two wall-connecting elements.

FIG. 12C illustrates a third, intramural cardiac anchor. In this case, the anchor is inserted into the ventricular wall **36,** from the external side, in a closed state. Once the anchor has reached the required depth within the ventricular wall, the wall-connecting element **48** is opened, by means of, for example, spring elements (not shown), thus embedding said wall-connecting element within the ventricular myocardium.

A particular advantage of the cardiac anchor connecting element is the fact that a series of such elements may be used to connect a plurality of *in vivo* devices (for example wire spring devices as described hereinabove) to the external ventricular wall. In this case, each of such spring devices is attached by its lateral ends to the heart by means of a pair of cardiac anchors. Each individual device will then be able to exert forces on its anchor pair, in such a way as to increase the linear separation distance between each member of said pair. Consequently, when the anchors are brought closer to each other during systolic movement of the ventricle, the spring will be compressed, thus storing potential energy. During diastole, this potential energy will be released as kinetic energy, thereby exerting radial expansive and tangential forces on the external wall of the filling ventricle. For example, the spring may be connected to its anchor pair during end diastole (when the left ventricle is filled to its greatest extent). During systolic contraction of the heart muscle, such a spring will be placed in a compressed state, absorbing potential energy, which in turn will be transformed into kinetic energy during the diastolic phase, thereby assisting in the filling of the left ventricle.

A further advantage of the cardiac anchor element, as described hereinabove, is the fact that it permits the presently disclosed and claimed in vivo devices to be used with a range of different sized hearts, and/or in hearts with aberrant morphology. For example, if a coronary artery is found to be located in an unusual position which might otherwise interfere with the placement of an in vivo device, said device can be conveniently positioned away from said artery.

In addition to the anatomical flexibility which is acquired by the use of cardiac anchors as connecting elements, said anchors further permit the use of standard *in* vivo devices for treating ventricles that require either relatively small or relatively large diastolic-assisting forces to be exerted thereon. This is achieved by varying the number of cardiac anchors attached to the ventricular wall, thereby permitting flexibility in the number of spring devices that may be anchored therein. Due to the ease with which the anchors and spring devices may be added, it is possible to continuously monitor the effect of the device on ventricular pressure changes, and to alter the number of springs used in response to said monitoring.

Another advantage of the cardiac anchors described herein is the fact that, due to their small size and elongated shape, they may be easily inserted into an endoscopic delivery mechanism, thus enabling the insertion of the in vivo device by use of minimally-invasive methods.

A further, significant, advantage of the use of cardiac anchors as the connecting means for the in *vivo* devices is related to the fact that said anchors may be attached to the ventricular wall in various different geometries. Typically, a line of such cardiac anchors may be arranged in a horizontally-disposed line, thus exerting tangential forces on the ventricular wall in a horizontal direction. However, if so required, the anchors may be so attached such that the device is orientated in other directions, thus permitting said device to exert tangential forces in said other directions, in accordance with individual clinical requirements.

The following non-limiting working example illustrates the insertion and use of the in vivo device of the present invention in a healthy mammalian subject.

### EXAMPLE

### IN VIVO DEMONSTRATION OF THE IMPLANTATION AND LOSE OF VARIOUS DEVICES IN A MAMMALIAN SUBJECT

### Method

### Anesthesia and Instrumentation:

A healthy sheep, (12 month, 31 Kg) was anesthetized (induction with xylazine + ketamine + valium; intubation and maintenance of anesthesia with enflurane; monitoring with ECG and saturation). A left thoracotomy incision was made and the chest was entered through the 5^{th} intercostal space. The pericardium was opened widely to allow access to the left ventricle. A fluid filled catheter was inserted into the left ventricle via the left atrial appendage and mitral valve, to allow continuous left ventricular pressure measurement and data acquisition to a PC. The distance from the base to the apex was 5-6 cm.

### Preparation for Device Attachment:

After recording stable LV pressures, three segments of 8mm diameter Dacron tube-grafts (3 cm-long each) were sutured to the LV free wall, using multiple interrupted stitches of 5/0 prolene. One segment was placed just left and parallel to the LAD coronary artery, avoiding a large diagonal branch; another segment was placed parallel to the PDA coronary artery (on its LV aspect) and the third segment was sutured midway between the two previous segments, ensuring that no damage was done to a large marginal branch of the CX coronary artery. The basal end of each graft was set approximately 1.5 cm from the AV groove, whereas the apical end was set approximately 1 cm from the apex. The heart was allowed to recover from the surgical manipulations and stable hemodynamics were achieved, with normal LV pressures.

### Device Attachment and Testing:

Before the insertion of each device into the three Dacron tubes, stable LV pressures were recorded. Pressure data was recorded again after the placement of the device within its tubes (after stabilization), and repeated after device removal. Eight different wire spring devices were tested in separate experiments.

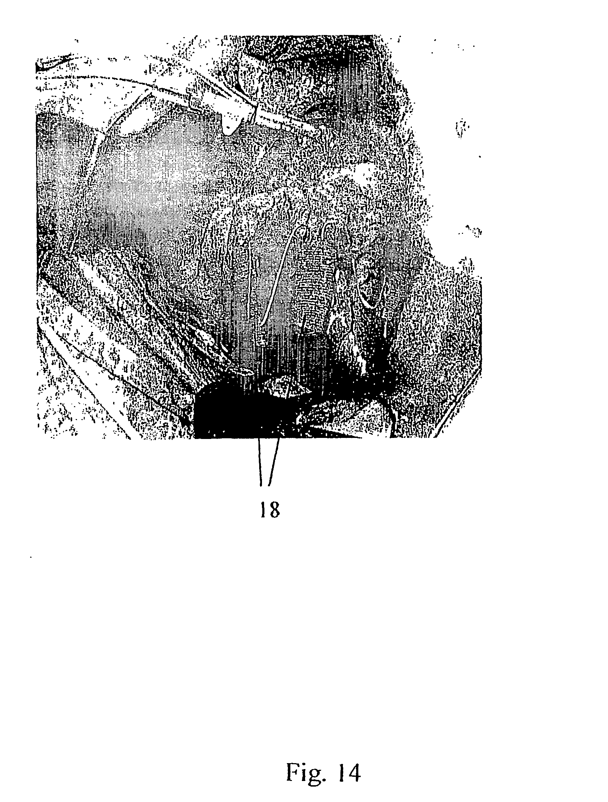

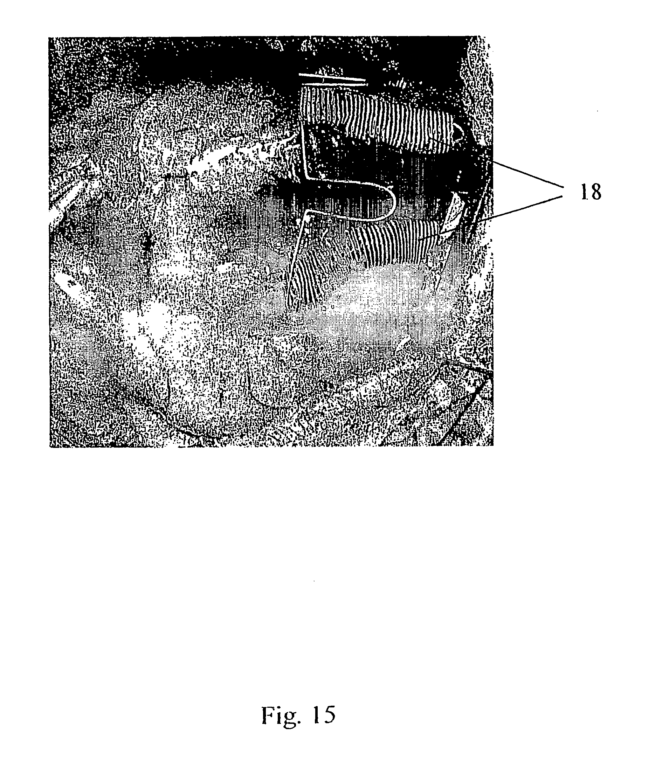

FIG. 14 and FIG. 15 demonstrate two different devices attached to the left ventricular wall. In each case, the in vivo device is shown attached to the external wall of the LV by means of the Dacron tubes 18.

### Results:

There was some patchy discoloration of the LV free wall after suturing of the Dacron tubes. However, this was transient and did not interfere with systolic blood pressure and parameters of cardiac output such as peripheral perfusion and urinary output.

Nine applications of 8 different devices, of various designs and elastic forces, were tested (device # 1 was tested twice). The cumulative time in which different devices were attached to the LV surface was approximately 90 minutes, and the changing of devices required multiple manipulations on the Dacron tubes. Despite these interventions the tubes remained attached firmly throughout the experiment. It should be noted that systolic LV pressure was not impaired by any of the devices tested (data not shown). Clinical parameters of perfusion were also satisfactory throughout the experiment.

## Claims

1. An anatomically-compatible and physiologically-compatible *in vivo* device for improving diastolic function of either the left or right ventricle of the heart, comprising
an elastic component (10) that is capable of being operatively connected to the left or right ventricle of the heart,
wherein said elastic component comprises a plurality of essentially elongated members (12), and wherein said essentially elongated members (12) are arranged relative to each other such that said elastic component is curved in both the vertical and horizontal planes, wherein:
said elastic component (10) is capable of being operatively connected to the external surface of said left or right ventricles by means of one or more connecting elements;
said elastic component is curved such that its inner surface may be adapted to the curvature of the external ventricular surface of the heart, or a portion thereof,
such that said at least one elastic component (10) is capable of exerting both a radially outward expansive force and a tangentially-directed force on the external surface of the ventricle to which said component may be connected by means of said one or more connecting elements;
said elongated members (12) are arranged such that they are capable of being disposed in an essentially longitudinal manner along the external ventricular surface of the heart, such that the free ends of said elongated members are directed towards the base of the heart;
said elongated members (12) are arranged such that the lateral separation between adjacent elongated members may be increased or decreased in response to elastic deformation of said elastic component,
**characterized in that**:
each of said elongated members (12) having one end connected to, and continuous with, a base element (14) and the other end being a free end, wherein said base element (14) being of a size and shape such that it is capable of either fully or partially encircling the apical region of the heart.

2. The device according to claim 1, wherein the base element is a curved base having a flat unfolded state.

3. The device according to claim 1, wherein the elongated members are perforated by a series of small holes in order to allow attachment thereof to the ventricular wall.

4. The device according to claim 1, wherein the base element is provided in an annular shape.

5. The device according to any one of claims 1 to 4, wherein the base element is self-expanding.

6. The device according to claim 2, wherein the angle formed between the first and last elongated members of the base element in the flat unfolded state is approximately 120°.

7. The device according to claim 1, wherein said device comprises two or more elastic components.

8. The device according to claim 2, wherein the angle between any two elongated members is about 30°.

9. The device according to any one of claims 1 to 8, wherein the longitudinal length of said device is in the range of 0.5 to 10 cm.

10. The device according to claim 7, wherein the at least one elastic component is constructed from a material selected from the group consisting of tungsten, platinum, titanium, nitinol alloy, stainless steel alloy, biocompatible plastics and, combinations thereof.

11. The device according to any one of claims 1 to 10, wherein the diameter across the widest end of said device is in the range of 0.1 to 6 cm.

12. The device according to any one of claims 1 to 11, wherein the thickness of the device is in the range of 0.01 to 5 mm.

## Patentansprüche

1. Anatomisch kompatible und physiologisch kompatible in-vivo-Vorrichtung zur Verbesserung der diastolischen Funktion entweder des linken oder rechten Ventrikels des Herzens, mit
einer elastischen Komponente (10), die imstande ist, wirksam mit dem linken oder rechten Ventrikel des Herzens verbunden zu werden,
wobei die elastische Komponente mehrere im wesentlichen längliche Elemente (12) aufweist, und wobei die im wesentlichen länglichen Elemente (12) relativ zueinander so angeordnet sind, dass die elastische Komponente sowohl in einer vertikalen als auch horizontalen Ebene gekrümmt ist, wobei:
die elastische Komponente (10) imstande ist, mittels eines oder mehrerer Verbindungselemente wirksam mit der Außenfläche des linken oder rechten Ventrikels verbunden zu sein;
die elastische Komponente so gekrümmt ist, dass ihre Innenfläche an die Krümmung der Ventrikelaußenfläche des Herzens oder eines Abschnitts davon angepasst werden kann,
so dass die mindestens eine elastische Komponente (10) imstande ist, sowohl eine radial nach außen gerichtete expansive Kraft und eine tangential gerichtete Kraft auf die Auβenfläche des Ventrikels auszuüben, mit dem die Komponente mittels des einen oder
der mehreren Verbindungselemente verbunden werden kann;
die länglichen Elemente (12) so angeordnet sind, dass sie imstande sind, in einer im wesentlichen longitudinalen Weise längs der Ventrikelaußenfläche des Herzens angeordnet zu werden, so dass die freien Enden der länglichen Elemente zur Basis des Herzens gerichtet sind;
die länglichen Elemente (12) so angeordnet sind, dass der laterale Abstand zwischen benachbarten länglichen Elemente als Reaktion auf eine elastische Verformung der elastischen Komponente erhöht oder gesenkt werden kann,
**dadurch gekennzeichnet, dass**:
jedes der länglichen Elemente (12) ein Ende aufweist, das mit einem Basiselement (14) verbunden und zusammenhängend ist, und das andere Ende ein freies Ende ist, wobei das Basiselement (14) eine solche Größe und Form aufweist, dass es imstande ist, entweder vollständig oder teilweise den Spitzenbereich des Herzens zu umgeben.

2. Vorrichtung nach Anspruch 1, wobei das Basiselement eine gekrümmte Basis ist, die einen ebenen entfalteten Zustand aufweist.

3. Vorrichtung nach Anspruch 1, wobei die länglichen Elemente durch eine Reihe von kleinen Löchern perforiert sind, um deren Befestigung an der Ventrikelwand zu ermöglichen.

4. Vorrichtung nach Anspruch 1, wobei das Basiselement in einer Ringform vorgesehen ist.

5. Vorrichtung nach einem der Ansprüche 1 bis 4, wobei das Basiselement selbstausdehnend ist.

6. Vorrichtung nach Anspruch 2, wobei der zwischen dem ersten und letzten länglichen Element des Basiselements im ebenen entfalteten Zustand ausgebildete Winkel annähernd 120° beträgt.

7. Vorrichtung nach Anspruch 1, wobei die Vorrichtung zwei oder mehrere elastische Komponenten aufweist.

8. Vorrichtung nach Anspruch 2, wobei der Winkel zwischen irgendwelchen zwei länglichen Elementen etwa 30° beträgt.

9. Vorrichtung nach einem der Ansprüche 1 bis 8, wobei die longitudinale Länge der Vorrichtung im Bereich von 0,5 bis 10 cm liegt.

10. Vorrichtung nach Anspruch 7, wobei die mindestens eine elastische Komponente aus einem Material aufgebaut ist, das aus der Gruppe ausgewählt ist, die aus Wolfram, Platin, Titan, einer Nitinol-Legierung, einer Edelstahllegierung, einem biokompatiblen Kunststoff und Kombinationen davon besteht.

11. Vorrichtung nach einem der Ansprüche 1 bis 10, wobei der Durchmesser am breitesten Ende der Vorrichtung im Bereich von 0,1 bis 6 cm liegt.

12. Vorrichtung nach einem der Ansprüche 1 bis 11, wobei die Dicke der Vorrichtung im Bereich von 0,01 bis 5 mm beträgt.

## Revendications

1. Dispositif *in vivo* anatomiquement compatible et physiologiquement compatible, destiné à améliorer la fonction diastolique du ventricule gauche ou du ventricule droit du coeur, comprenant :
un composant élastique (10) pouvant être fonctionnellement connecté au ventricule gauche ou au ventricule droit du coeur,
ledit ou lesdits composants élastiques comprenant une pluralité d'éléments sensiblement oblongs (12), lesdits éléments sensiblement oblongs (12) étant disposés les uns par rapport aux autres de manière à incurver le composant élastique sur le plan horizontal ainsi que sur le plan vertical, et où :
le composant élastique (10) peut être fonctionnellement connecté à la surface extérieure du ventricule gauche ou du ventricule droit au moyen d'un ou de plusieurs éléments de connexion ;
le composant élastique est incurvé de telle manière que sa surface intérieure peut être ajustée à la courbe de la surface ventriculaire extérieure du coeur, ou à une partie de celle-ci,
de telle manière que le ou les composants élastiques (10) peuvent exercer un effort d'expansion radiale vers l'extérieur ainsi qu'un effort à direction tangentielle sur la surface extérieure du ventricule à laquelle ledit composant peut être connecté au moyen du ou des éléments de connexion ;
les éléments oblongs (12) étant prévus de manière à pouvoir être disposés de manière sensiblement longitudinale le long de la surface ventriculaire extérieure du coeur, si bien que les extrémités libres desdits éléments oblongs sont dirigées vers la base du coeur ;
les éléments oblongs (12) étant disposés de telle manière que la séparation latérale entre des éléments oblongs adjacents puisse être augmentée ou diminuée en réaction à une déformation élastique du composant élastique,
**caractérisé en ce que** :
chaque élément oblong (12) a une extrémité reliée à, et dans le prolongement d'un élément de base (14), et **en ce que** l'autre extrémité est une extrémité libre, l'élément de base (14) étant de dimension et de forme lui permettant d'entourer complètement ou
partiellement la région apicale du coeur.

2. Dispositif selon la revendication 1, où l'élément de base est une base incurvée en état plat non plié.

3. Dispositif selon la revendication 1, où les éléments oblongs sont perforés d'une série de petits trous pour permettre leur fixation à la paroi ventriculaire.

4. Dispositif selon la revendication 1, où l'élément de base est prévu avec une forme annulaire.

5. Dispositif selon l'une des revendications 1 à 4, où l'élément de base est auto-expansible.

6. Dispositif selon la revendication 2, où l'angle formé entre le premier et le dernier éléments oblongs de l'élément de base en état plat non plié est sensiblement égal à 120 °.

7. Dispositif selon la revendication 1, où ledit dispositif comprend deux ou plusieurs composants élastiques.

8. Dispositif selon la revendication 2, où l'angle entre deux éléments oblongs quelconques est sensiblement égal à 30 °.

9. Dispositif selon l'une des revendications 1 à 8, où la longueur dans la direction longitudinale dudit dispositif est comprise entre 0,5 et 10 cm.

10. Dispositif selon la revendication 7, où le ou les composants élastiques sont constitués d'un matériau sélectionné dans le groupe composé de tungstène, platine, titane, alliage nitinol, alliage d'acier inoxydable, plastiques biocompatibles et des combinaisons de ceux-ci.

11. Dispositif selon l'une des revendications 1 à 10, où le diamètre de l'extrémité la plus large dudit dispositif est compris entre 0,1 et 6 cm.

12. Dispositif selon l'une des revendications 1 à 11, où l'épaisseur dudit dispositif est comprise entre 0,01 et 5 mm.
